(19)

Europäisches
Patentamt
European
Patent Office
Office européen
des brevets

(11) **EP 4 151 655 A1**

(12) **EUROPEAN PATENT APPLICATION**
published in accordance with Art. 153(4) EPC

(43) Date of publication:
**22.03.2023 Bulletin 2023/12**

(21) Application number: **21804918.7**

(22) Date of filing: **14.05.2021**

(51) International Patent Classification (IPC):
**C07K 16/28** $^{(2006.01)}$        **C07K 14/55** $^{(2006.01)}$
**A61K 39/00** $^{(2006.01)}$

(52) Cooperative Patent Classification (CPC):
**A61K 39/00; C07K 14/55; C07K 16/28**

(86) International application number:
**PCT/CN2021/093791**

(87) International publication number:
**WO 2021/228218 (18.11.2021 Gazette 2021/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.05.2020 CN 202010408502**

(71) Applicants:
• **Jiangsu Hengrui Pharmaceuticals Co., Ltd.
Lianyungang, Jiangsu 222047 (CN)**
• **Shanghai Shengdi Pharmaceutical Co., Ltd
Shanghai 201210 (CN)**

(72) Inventors:
• **CHEN, Simeng
Lianyungang, Jiangsu 222047 (CN)**

• **ZHANG, Wei
Lianyungang, Jiangsu 222047 (CN)**
• **JIANG, Fuwei
Lianyungang, Jiangsu 222047 (CN)**
• **LIAO, Cheng
Lianyungang, Jiangsu 222047 (CN)**

(74) Representative: **Potter Clarkson
Chapel Quarter
Chapel Bar
Nottingham NG1 6HQ (GB)**

Remarks:
The complete document including Reference
Table(s) and the Sequence Listing(s) can be
downloaded from the EPO website

(54) **ANTI-CD25 ANTIBODIES, ANTIGEN-BINDING FRAGMENTS THEREOF, AND MEDICAL USES THEREOF**

(57)    The present disclosure relates to anti-CD25 antibodies, antigen-binding fragments thereof, and medical uses thereof. Specifically, the present disclosure relates to anti-CD25 antibodies and their use for preparing anti-tumor drugs.

EP 4 151 655 A1

**Description**

The present application claims priority to Chinese Patent Application (Application No. 202010408502.3) filed on May. 14, 2020, which is incorporated herein by reference in its entirety.

**TECHNICAL FIELD**

The present disclosure belongs to the field of biomedicine, particularly to the field of cancer immunotherapy, including methods for treating cancer (e.g., solid tumors), and relates to the use of an anti-CD25 antibody or an antigen-binding fragment thereof.

**BACKGROUND**

Tumor immunotherapy is a hot spot in the field of tumor therapy, and is currently mainly achieved by increasing the number and activity of $CD4^+$ and $CD8^+$ T cells in the tumor or suppressing the number and activity of inhibitory immune cells in the tumor, e.g., suppressing regulatory T cells (Tregs), myeloid-derived suppressor cells (MDSCs) or tumor-associated macrophages. Therapeutic strategies by reducing or eliminating Treg cells in the tumor have been clinically validated.

CD25 is a type I membrane protein consisting of 272 amino acids, whose expression on T cells is regulated by TCR signaling, and therefore is often used as a marker for T cell activation. IL-2 has an immune-activating effect and is an immunomodulator in tumor therapy, which acts through the IL-2 receptor (IL-2R) on the cell surface. IL-2R includes three subunits: IL-2R$\alpha$ (i.e., CD25), IL-2R$\beta$ (i.e., CD122), and IL-2R$\gamma$ (i.e., CD132). Those three subunits can form three forms of receptors: the high-binding affinity receptor comprising all of the three subunits IL-2R$\alpha/\beta/\gamma$, the medium-binding affinity receptor comprising IL-2R$\beta/\gamma$, and the low-binding affinity receptor being IL-2R$\alpha$. IL-2R$\alpha$ (i.e., CD25) alone has a low affinity for IL-2 (Kd of $10^{-8}$ M), and does not transmit intracellular signals. IL-2R$\beta$ and IL-2R$\gamma$ are necessary for IL-2 to activate downstream signaling pathways, and when IL-2 binds to both IL-2R$\beta$ and IL-2R$\gamma$, the two receptor subunits form heterodimers. When IL-2R$\alpha$ (i.e., CD25) forms a high-affinity receptor (Kd of $10^{-11}$M) with IL-2R$\beta$ and IL-2R$\gamma$, the downstream JAK-STAT, PI3K-AKT and MAPK signal pathways can be activated, so that the proliferation and the activity of lymphocytes such as T cells and NK cells are promoted.

Some existing anti-CD25 antibodies block or inhibit the binding of IL-2 to CD25, see, e.g., WO2004/045512, WO2006/108670, WO1993/011238, WO1990/007861, and WO2017/174331. Both basiliximab and daclizumab (DAC) are anti-human CD25 antibodies of the IgG1 type that inhibit the binding of IL-2 to CD25, and have been developed to reduce the activation of effector T cells (Teffs) based on the mediation of immune activation function of IL-2 by CD25. Basiliximab is a chimeric anti-CD25 antibody currently approved for graft versus host disease (GVHD), while daclizumab is a humanized anti-CD25 antibody approved for the treatment of multiple sclerosis. Since the expression abundance of CD25 on Tregs is much higher than that of other immune cells, CD25 can also be used as a Treg-specific marker for developing antibodies, removing Treg cells in the tumor through antibody-dependent cell-mediated cytotoxicity (ADCC) and antibody-dependent cellular phagocytosis (ADCP), and relieving the inhibition of Tregs on T cells in the tumor, thereby promoting anti-tumor immunity. However, CD25 also mediates the activation of effector T cells by IL-2, and if the IL-2 signaling pathway is inhibited by the CD25 antibody, Teff will be inhibited and its anti-tumor activity will be antagonized. The use of anti-CD25 alone or in combination for cancer or in relation to Treg depletion is mentioned in some documents (WO 2004/074437, WO 2006/108670, WO 2006/050172, WO 2011/077245, WO 2016/021720, and WO 2004/045512), and therefore there is a need for an anti-CD25 antibody that allows the binding of IL-2 to CD25. Preclinically developed CD25 antibodies include 7D4 (rat anti-mouse CD25, see, e.g., Malek et al., PNAS, 1983 Sep; 80(18): 5694-5698; Onizuka S et al., CancerRes., 1999 Jul 1;59(13): 3128-3133), 7G7B6 (anti-human CD25, see, e.g., Zhang et al., Cancer Biother Radiopharm., 2009 Jun; 24(3): 303-309), PC61 (rat anti-mouse CD25, see, e.g., Yulius Y Setiady et al., Eur J Immunol. 2010 Mar; 40(3): 780-6). When tested in a mouse cancer model, PC61 fails to exhibit anti-tumor activity due to its inhibitory effect on the binding of CD25 to IL2, while 7D4 exhibits anti-tumor activity superior to PC61 because it does not affect the binding of IL2 to CD25. In addition, provided in WO2019/175216 is an antibody RG6292 targeting CD25 developed jointly by Roche and TUSK, which has been brought into clinical stage I and tested for its safety and efficacy in solid tumors. Also provided in WO2018167104 is an anti-CD25 antibody that does not affect the binding of IL-2 to CD25.

It is still an urgent need in the art to develop an anti-CD25 antibody with superior tumor suppression effect and higher safety, which does not inhibit the binding of IL-2 to CD25, and is capable of eliminating Tregs through ADCC and ADCP effects, while avoiding the inhibition of Teff activity.

## SUMMARY

The present disclosure provides an anti-CD25 antibody and an antigen-binding fragment thereof, and an encoding nucleic acid, a vector, a host cell, a pharmaceutical composition, a method for treating cancer (particularly solid tumors), and pharmaceutical use thereof.

Anti-CD25 Antibody or Antigen-Binding Fragment Thereof

The present disclosure provides an anti-CD25 antibody or an antigen-binding fragment thereof, which comprises a heavy chain variable region (VH) and/or a light chain variable region (VL), wherein:

1) the VH comprises an HCDR1, an HCDR2 and an HCDR3 in SEQ ID NO: 1, and the VL comprises an LCDR1, an LCDR2 and an LCDR3 in SEQ ID NO: 2;
2) the VH comprises an HCDR1, an HCDR2 and an HCDR3 in SEQ ID NO: 3, and the VL comprises an LCDR1, an LCDR2, and an LCDR3 in SEQ ID NO: 4;
3) the VH comprises an HCDR1, an HCDR2 and an HCDR3 in SEQ ID NO: 5, and the VL comprises an LCDR1, an LCDR2 and an LCDR3 in SEQ ID NO: 6;
4) the VH comprises an HCDR1, an HCDR2 and an HCDR3 in SEQ ID NO: 7, and the VL comprises an LCDR1, an LCDR2 and an LCDR3 in SEQ ID NO: 8;
5) the VH comprises an HCDR1, an HCDR2 and an HCDR3 in SEQ ID NO: 9, and the VL comprises an LCDR1, an LCDR2, and an LCDR3 in SEQ ID NO: 10;
6) the VH comprises an HCDR1, an HCDR2 and an HCDR3 in SEQ ID NO: 11, and the VL comprises an LCDR1, an LCDR2 and an LCDR3 in SEQ ID NO: 12;
7) the VH comprises an HCDR1, an HCDR2 and an HCDR3 in SEQ ID NO: 13, and the VL comprises an LCDR1, an LCDR2, and an LCDR3 in SEQ ID NO: 14;
8) the VH comprises an HCDR1, an HCDR2 and an HCDR3 in SEQ ID NO: 15, and the VL comprises an LCDR1, an LCDR2 and an LCDR3 in SEQ ID NO: 16;
9) the VH comprises an HCDR1, an HCDR2 and an HCDR3 in SEQ ID NO: 17, and the VL comprises an LCDR1, an LCDR2 and an LCDR3 in SEQ ID NO: 18;
10) the VH comprises an HCDR1, an HCDR2 and an HCDR3 in SEQ ID NO: 19, and the VL comprises an LCDR1, an LCDR2 and an LCDR3 in SEQ ID NO: 20;
11) the VH comprises an HCDR1, an HCDR2 and an HCDR3 in SEQ ID NO: 21, and the VL comprises an LCDR1, an LCDR2 and an LCDR3 in SEQ ID NO: 22;
12) the VH comprises an HCDR1, an HCDR2 and an HCDR3 in SEQ ID NO: 23, and the VL comprises an LCDR1, an LCDR2, and an LCDR3 in SEQ ID NO: 24;
13) the VH comprises an HCDR1, an HCDR2 and an HCDR3 in SEQ ID NO: 25, and the VL comprises an LCDR1, an LCDR2 and an LCDR3 in SEQ ID NO: 26; or
14) the VH comprises an HCDR1, an HCDR2 and an HCDR3 in SEQ ID NO: 59, and the VL comprises an LCDR1, an LCDR2 and an LCDR3 in SEQ ID NO: 60.

The above CDRs are defined according to the Kabat, IMGT, Chothia, AbM or Contact numbering scheme; in some specific embodiments, the CDRs are defined according to the Kabat numbering scheme.
In some embodiments, the antibody or the antigen-binding fragment thereof comprising a VH and a VL as shown in 1), 2), 3), 5), or 6) blocks or partially blocks the binding of IL-2 to CD25; in other embodiments, the antibody or the antigen-binding fragment thereof comprising a VH and a VL as shown in 4), 7), 8), 9), 10), 11), 12), or 13) does not block or inhibit, or hardly blocks or inhibits the binding of IL-2 to CD25.
In some embodiments, provided is an anti-CD25 antibody or an antigen-binding fragment thereof, which comprises a VH and/or a VL, wherein:
the VH comprises an HCDR1 selected from the group consisting of SEQ ID NOs: 27, 33, 39 and 45, and/or an HCDR2 selected from the group consisting of SEQ ID NOs: 28, 34, 40 and 46, and/or an HCDR3 selected from the group consisting of SEQ ID NOs: 29, 35, 41 and 47; the VL comprises an LCDR1 selected from the group consisting of SEQ ID NOs: 30, 36, 42 and 48, and/or an LCDR2 selected from the group consisting of SEQ ID NOs: 31, 37, 43 and 49, and/or an LCDR3 selected from the group consisting of SEQ ID NOs: 32, 38, 44 and 50.
In some embodiments, provided is an anti-CD25 antibody or an antigen-binding fragment thereof, which comprises a VH and/or a VL, wherein:

i) the VH comprises an HCDR1 set forth in SEQ ID NO: 27 or having at most 3, 2 or 1 amino acid mutation compared thereto, an HCDR2 set forth in SEQ ID NO: 28 or having at most 3, 2 or 1 amino acid mutation compared thereto, and an HCDR3 set forth in SEQ ID NO: 29 or having at most 3, 2 or 1 amino acid mutation compared thereto; and/or the VL comprises an LCDR1 set forth in SEQ ID NO: 30 or having at most 3, 2 or 1 amino acid mutation compared thereto, an LCDR2 set forth in SEQ ID NO: 31 or having at most 3, 2 or 1 amino acid mutation compared thereto, and an LCDR3 set forth in SEQ ID NO: 32 or having at most 3, 2 or 1 amino acid mutation compared thereto;

ii) the VH comprises an HCDR1 set forth in SEQ ID NO: 33 or having at most 3, 2 or 1 amino acid mutation compared thereto, an HCDR2 set forth in SEQ ID NO: 34 or having at most 3, 2 or 1 amino acid mutation compared thereto, and an HCDR3 set forth in SEQ ID NO: 35 or having at most 3, 2 or 1 amino acid mutation compared thereto; and/or the VL comprises an LCDR1 set forth in SEQ ID NO: 36 or having at most 3, 2 or 1 amino acid mutation compared thereto, an LCDR2 set forth in SEQ ID NO: 37 or having at most 3, 2 or 1 amino acid mutation compared thereto, and an LCDR3 set forth in SEQ ID NO: 38 or having at most 3, 2 or 1 amino acid mutation compared thereto;

iii) the VH comprises an HCDR1 set forth in SEQ ID NO: 39 or having at most 3, 2 or 1 amino acid mutation compared thereto, an HCDR2 set forth in SEQ ID NO: 40 or having at most 3, 2 or 1 amino acid mutation compared thereto, and an HCDR3 set forth in SEQ ID NO: 41 or having at most 3, 2 or 1 amino acid mutation compared thereto; and/or the VL comprises an LCDR1 set forth in SEQ ID NO: 42 or having at most 3, 2 or 1 amino acid mutation compared thereto, an LCDR2 set forth in SEQ ID NO: 43 or having at most 3, 2 or 1 amino acid mutation compared thereto, and an LCDR3 set forth in SEQ ID NO: 44 or having at most 3, 2 or 1 amino acid mutation compared thereto; or

iv) the VH comprises an HCDR1 set forth in SEQ ID NO: 45 or having at most 3, 2 or 1 amino acid mutation compared thereto, an HCDR2 set forth in SEQ ID NO: 46 or having at most 3, 2 or 1 amino acid mutation compared thereto, and an HCDR3 set forth in SEQ ID NO: 47 or having at most 3, 2 or 1 amino acid mutation compared thereto; and/or the VL comprises an LCDR1 set forth in SEQ ID NO: 48 or having at most 3, 2 or 1 amino acid mutation compared thereto, an LCDR2 set forth in SEQ ID NO: 49 or having at most 3, 2 or 1 amino acid mutation compared thereto, and an LCDR3 set forth in SEQ ID NO: 50 or having at most 3, 2 or 1 amino acid mutation compared thereto.

In some specific embodiments, the above anti-CD25 antibody or antigen-binding fragment thereof having amino acid mutations has identical or substantially identical affinity and/or function (e.g., ADCC, ADCP or anti-tumor activity) for binding to human CD25 to the parent antibody or the antigen-binding fragment.

In some embodiments, the above anti-CD25 antibody or the antigen-binding fragment thereof of the present disclosure binds to human CD25 with a dissociation equilibrium constant equal to or less than $10^{-7}$ M. In some embodiments, the above anti-CD25 antibody or the antigen-binding fragment thereof of the present disclosure binds to human CD25 with a dissociation equilibrium constant equal to or less than $10^{-8}$ M, $10^{-9}$ M, $10^{-10}$ M, or $10^{-11}$ M.

In some embodiments, provided is an anti-CD25 antibody or an antigen-binding fragment thereof, which comprises a VH and/or a VL, wherein:

the VH comprises an HCDR1, an HCDR2 and an HCDR3 set forth in SEQ ID NOs: 27, 28 and 29, respectively, and the VL comprises an LCDR1, an LCDR2 and an LCDR3 set forth in SEQ ID NOs: 30, 31 and 32, respectively;
the VH comprises an HCDR1, an HCDR2 and an HCDR3 set forth in SEQ ID NOs: 33, 34 and 35, respectively, and the VL comprises an LCDR1, an LCDR2 and an LCDR3 set forth in SEQ ID NOs: 36, 37 and 38, respectively;
the VH comprises an HCDR1, an HCDR2 and an HCDR3 set forth in SEQ ID NOs: 39, 40 and 41, respectively, and the VL comprises an LCDR1, an LCDR2 and an LCDR3 set forth in SEQ ID NOs: 42, 43 and 44, respectively; or
the VH comprises an HCDR1, an HCDR2 and an HCDR3 set forth in SEQ ID NOs: 45, 46 and 47, respectively, and the VL comprises an LCDR1, an LCDR2 and an LCDR3 set forth in SEQ ID NOs: 48, 49 and 50, respectively.

In some embodiments, the anti-CD25 antibody or the antigen-binding fragment thereof comprises a VH and a VL, wherein:

the VH is set forth in SEQ ID NO: 1, and the VL is set forth in SEQ ID NO: 2;
the VH is set forth in SEQ ID NO: 3, and the VL is set forth in SEQ ID NO: 4;
the VH is set forth in SEQ ID NO: 5, and the VL is set forth in SEQ ID NO: 6;
the VH is set forth in SEQ ID NO: 7, and the VL is set forth in SEQ ID NO: 8;
the VH is set forth in SEQ ID NO: 9, and the VL is set forth in SEQ ID NO: 10;
the VH is set forth in SEQ ID NO: 11, and the VL is set forth in SEQ ID NO: 12;
the VH is set forth in SEQ ID NO: 13, and the VL is set forth in SEQ ID NO: 14;
the VH is set forth in SEQ ID NO: 15, and the VL is set forth in SEQ ID NO: 16; or
the VH is set forth in SEQ ID NO: 17, and the VL is set forth in SEQ ID NO: 18; the antibody is a murine antibody or a fragment thereof.

In some specific embodiments, provided is a variant of an anti-CD25 antibody or an antigen-binding fragment thereof, which comprises a VH and/or a VL, wherein the VH and/or VL have at least 80%, 85%, 90%, 95%, 96%, 97%, 98%, or 99% sequence identity to the VH and/or VL of the above anti-CD25 antibody or the antigen-binding fragment thereof, respectively.

In some embodiments, the anti-CD25 antibody or the antigen-binding fragment thereof is a murine antibody, a chimeric antibody, a human antibody, or a humanized antibody or a fragment thereof, e.g., a humanized antibody or a fragment thereof.

In some embodiments, the chimeric antibody is prepared based on the murine anti-CD25 antibody or the antigen-binding fragment thereof, humanized, and then back-mutated. The methods of humanization in US20030040606 and US7494647 are incorporated herein in their entirety.

In some embodiments, the anti-CD25 antibody or the antigen-binding fragment thereof comprises a VH and a VL, wherein:

the VH comprises an FR1 to an FR3 selected from IGHV1-46*01, and an FR4 selected from IGHJ1*01, and the VL comprises an FR1 to an FR3 selected from IGKV4-1*01, and an FR4 selected from IGKJ4*01;

the VH comprises an FR1 to an FR2 selected from IGHV1-18*01, an FR3 selected from IGHV1-69*02, and an FR4 selected from hIGHJ6*01_14, and the VL comprises an FR1 selected from IGKV3-11*01, an FR2 selected from IGKV5-2*01, an FR3 selected from IGKV6-21*01, and an FR4 selected from hIGKJ4*01_12;

the VH comprises an FR1 selected from IGHV1-18*01, an FR2 selected from IGHV4-31*01, an FR3 selected from IGHV1-3*01, and an FR4 selected from hIGHJ6*01, and the VL comprises an FR1 to an FR3 selected from IGKV4-1*01, and an FR4 selected from hIGKJ2*01; or

the VH comprises an FR1 to an FR3 selected from IGHV3-23*04, and an FR4 selected from IGHJ1*01, and the VL comprises an FR1 to an FR3 selected from IGKV2-28*01, and an FR4 selected from IGKJ4*01.

In some specific embodiments, the anti-CD25 antibody or the antigen-binding fragment thereof comprises a VH and/or a VL, wherein,

the VH comprises an HCDR1, an HCDR2 and an HCDR3 set forth in SEQ ID NOs: 27, 28 and 29, respectively, and an FR1 to an FR3 selected from IGHV1-46*01, and an FR4 selected from IGHJ1*01; the VL comprises an LCDR1, an LCDR2 and an LCDR3 set forth in SEQ ID NOs: 30, 31 and 32, respectively, and an FR1 to an FR3 selected from IGKV4-1*01, and an FR4 selected from IGKJ4*01;

the VH comprises an HCDR1, an HCDR2 and an HCDR3 set forth in SEQ ID NOs: 33, 34 and 35, respectively, and an FR1 to an FR2 selected from IGHV1-18*01, an FR3 selected from IGHV1-69*02, and an FR4 selected from hIGHJ6*01_14; the VL comprises an LCDR1, an LCDR2 and an LCDR3 set forth in SEQ ID NOs: 36, 37 and 38, respectively, and an FR1 selected from IGKV3-11*01, an FR2 selected from IGKV5-2*01, an FR3 selected from IGKV6-21*01, and an FR4 selected from hIGKJ4*01_12;

the VH comprises an HCDR1, an HCDR2 and an HCDR3 set forth in SEQ ID NOs: 39, 40 and 41, respectively, and an FR1 selected from IGHV1-18*01, an FR2 selected from IGHV4-31*01, an FR3 selected from IGHV1-3*01, and an FR4 selected from hIGHJ6*01; the VL comprises an LCDR1, an LCDR2 and an LCDR3 set forth in SEQ ID NOs: 42, 43 and 44, and an FR1 to an FR3 selected from IGKV4-1*01, and an FR4 selected from hIGKJ2*01; or

the VH comprises an HCDR1, an HCDR2 and an HCDR3 set forth in SEQ ID NOs: 45, 46 and 47, respectively, and an FR1 to an FR3 selected from IGHV3-23*04, and an FR4 selected from IGHJ1*01; the VL comprises an LCDR1, an LCDR2 and an LCDR3 set forth in SEQ ID NOs: 48, 49 and 50, respectively, and an FR1 to an FR3 selected from IGKV2-28*01, and an FR4 selected from IGKJ4*01. The above FRs can be back-mutated to retain the antibody activity.

In some embodiments, the anti-CD25 antibody or the antigen-binding fragment thereof comprises a VH and/or a VL, wherein:

the VH is set forth in SEQ ID NO: 19, and the VL is set forth in SEQ ID NO: 20;
the VH is set forth in SEQ ID NO: 21, and the VL is set forth in SEQ ID NO: 22;
the VH is set forth in SEQ ID NO: 23, and the VL is set forth in SEQ ID NO: 24;
the VH is set forth in SEQ ID NO: 25, and the VL is set forth in SEQ ID NO: 26; or the VH is set forth in SEQ ID NO: 59, and the VL is set forth in SEQ ID NO: 60; the antibody is a humanized antibody or a fragment thereof.

In some specific embodiments, provided is a variant of an anti-CD25 antibody or an antigen-binding fragment thereof, which comprises a VH and/or a VL, wherein the VH and/or VL have at least 80%, 85%, 90%, 95%, 96%, 97%, 98%, or 99% sequence identity to the VH and/or VL of the above anti-CD25 antibody or the antigen-binding fragment thereof, respectively.

In some embodiments, the anti-CD25 antibody or the antigen-binding fragment thereof further comprises a heavy chain constant region and/or a light chain constant region of an antibody, for example, a heavy chain constant region of human IgG1, IgG2, IgG3 and IgG4 and conventional variants thereof, and a light chain constant region selected from the group consisting of κ and λ chain constant regions of a human antibody and conventional variants thereof; for another example, murine IgG, such as murine IgG2a. In some embodiments, the antigen-binding fragment is selected from the group consisting of Fab, Fab', F(ab')$_2$, a single chain antibody (scFv), a dimerized V region (diabody), a disulfide-stabilized V region (dsFv), and an antigen-binding fragment of other CDR-containing peptides.

In some embodiments, also provided is an isolated monoclonal antibody or an antigen-binding fragment thereof, which competes for binding to human CD25, or for binding to the same epitope, with the anti-CD25 antibody or the antigen-binding fragment thereof according to any one of the above embodiments.

In some embodiments, provided is an anti-CD25 antibody or an antigen-binding fragment thereof, which comprises a VH and/or a VL, wherein:

the VH is set forth in SEQ ID NO: 1, and the VL is set forth in SEQ ID NO: 2;
the VH is set forth in SEQ ID NO: 3, and the VL is set forth in SEQ ID NO: 4;
the VH is set forth in SEQ ID NO: 5, and the VL is set forth in SEQ ID NO: 6;
the VH is set forth in SEQ ID NO: 9, and the VL is set forth in SEQ ID NO: 10; or the VH is set forth in SEQ ID NO: 11, and the VL is set forth in SEQ ID NO: 12; the antibody or the antigen-binding fragment thereof blocks or partially blocks the binding of IL-2 to CD25.

In some other embodiments, provided is an anti-CD25 antibody or an antigen-binding fragment thereof, which comprises a VH and/or a VL, wherein:

the VH is set forth in SEQ ID NO: 7, and the VL is set forth in SEQ ID NO: 8;
the VH is set forth in SEQ ID NO: 13, and the VL is set forth in SEQ ID NO: 14;
the VH is set forth in SEQ ID NO: 15, and the VL is set forth in SEQ ID NO: 16;
the VH is set forth in SEQ ID NO: 17, and the VL is set forth in SEQ ID NO: 18;
the VH is set forth in SEQ ID NO: 19, and the VL is set forth in SEQ ID NO: 20;
the VH is set forth in SEQ ID NO: 21, and the VL is set forth in SEQ ID NO: 22;
the VH is set forth in SEQ ID NO: 23, and the VL is set forth in SEQ ID NO: 24;
the VH is set forth in SEQ ID NO: 25, and the VL is set forth in SEQ ID NO: 26; or
the VH is set forth in SEQ ID NO: 59, and the VL is set forth in SEQ ID NO: 60; the antibody or the antigen-binding fragment thereof does not block or inhibit, hardly blocks or inhibits, or to a lesser extent, blocks or inhibits the binding of IL-2 to CD25. For example, the anti-CD25 antibody or the antigen-binding fragment blocks IL-2 signaling by less than about 50%, about 40%, about 35%, about 30%, about 25%, about 20%, about 15%, or about 10% of IL-2 signaling, e.g., less than about 25%, as compared to IL-2 signaling in the absence of the antibody.

In some embodiments, provided is a variant of an anti-CD25 antibody or an antigen-binding fragment thereof, which comprises a VH and/or a VL, wherein the VH and/or VL have at least 80%, 85%, 90%, 95%, 96%, 97%, 98%, or 99% sequence identity to the VH and/or VL of the above anti-CD25 antibody or the antigen-binding fragment thereof.

In some embodiments, provided is a variant of an anti-CD25 antibody or an antigen-binding fragmentthereof, which comprises a VH and/or a VL, wherein the VH and/or the VL each comprise 0, 1, 2, 3, 4, 5, 6, 7, 8, 9 or 10 amino acid changes, as compared to the heavy chain variable region VH and/or VL of the above anti-CD25 antibody or the antigen-binding fragment thereof. The amino acid change may be a conservative substitution of an amino acid residue in the variable region. In some embodiments, the above antibody or the antigen-binding fragment comprising amino acid changes has identical or substantially identical affinity and/or function (e.g., ADCC, ADCP or anti-tumor activity) for binding to human CD25 to the parent antibody or the antigen-binding fragment.

In some embodiments, the anti-CD25 antibody or the antigen-binding fragment thereof comprises a heavy chain and/or a light chain, wherein,

the heavy chain is set forth in SEQ ID NO: 51, and the light chain is set forth in SEQ ID NO: 52;
the heavy chain is set forth in SEQ ID NO: 53, and the light chain is set forth in SEQ ID NO: 54;
the heavy chain is set forth in SEQ ID NO: 55, and the light chain is set forth in SEQ ID NO: 56;
the heavy chain is set forth in SEQ ID NO: 57, and the light chain is set forth in SEQ ID NO: 58; or
the heavy chain is set forth in SEQ ID NO: 61, and the light chain is set forth in SEQ ID NO: 62.

In some embodiments, provided is an anti-CD25 antibody or an antigen-binding fragment thereof, which comprises a heavy chain and/or a light chain, wherein the heavy chain and/or the light chain have at least 70%, 80%, 85%, 90%, 95%, 96%, 97%, 98%, or 99% sequence identity to the heavy chain and/or the light chain of the above anti-CD25 antibody or the antigen-binding fragment thereof.

In some embodiments, the anti-CD25 antibody or the antigen-binding fragment thereof of the present disclosure is of the IgG1 type, with a de-fucosylated site in the Fc region and enhanced FcγRIIIa-binding ability. The above anti-CD25 antibody or the antigen-binding fragment thereof can increase an ADCC effect on Treg cells and enhance the anti-tumor activity of the antibody.

In some embodiments, the anti-CD25 antibody or the antigen-binding fragment thereof of the present disclosure is of the IgG1 type, with a de-fucosylated site in the Fc region (e.g., A330I mutation) and reduced FcγRIIb-binding ability. The above anti-CD25 antibody or the antigen-binding fragment thereof can reduce FcγRIIb receptor-mediated inhibitory signals to ADCC/ADCP, so as to enhance an ADCC effect on Treg cells and enhance the anti-tumor activity of the antibody.

In some embodiments, the anti-CD25 antibody or the antigen-binding fragment of the present disclosure has at least one of the following characteristics:

(a) having a KD value for binding to human CD25 of less than $1 \times 10^{-7}$ M;
(b) having no inhibition (or substantially no inhibition) on the binding of IL-2 to CD25;
(c) depleting tumor-infiltrating Tregs, without affecting (or substantially without affecting) Teff function;
(d) binding to an Fcγ receptor with an activation/inhibition (A/I) rate greater than 1;
(e) binding to FcγRIIIa with higher affinity than binding to FcγRI, FcγRIIc and/or FcγRIIb, and binding to FcγRIIIa with higher affinity than binding to FcγRIIb; and
(f) inhibiting the growth of various tumors.

The CD25-binding protein or the anti-CD25 antibody of the present disclosure may have a KD value for binding to CD25 of less than $1 \times 10^{-7}$ M, less than $1 \times 10^{-8}$ M, less than $1 \times 10^{-9}$ M, or less than $1 \times 10^{-10}$ M.

The anti-CD25 antibody or the antigen-binding fragment thereof of the present disclosure can inhibit tumor growth by at least about 10%, at least about 20%, at least about 30%, at least about 40%, at least about 50%, at least about 60%, at least about 70% or at least about 80%.

In some embodiments, the anti-CD25 antibody or the antigen-binding fragment of the present disclosure binds to FcγR with high affinity, e.g., bind to an activating receptor with high affinity. In some specific embodiments, the antibody of the present disclosure binds to FcγRI and/or FcγRIIA and/or FcγRIIIA with high affinity. In specific embodiments, the antibody binds to at least one activating Fcγ receptor with a dissociation constant of less than about $10^{-6}$ M, $10^{-7}$ M, $10^{-8}$ M, $10^{-9}$ M, or $10^{-10}$ M.

In some embodiments, the anti-CD25 antibody or the antigen-binding fragment of the present disclosure is an IgG1 antibody, which can bind to at least one Fc activating receptor. For example, the antibody can bind to one or more receptors selected from the group consisting of FcγRI, FcγRIIa, FcγRIIc, FcγRIIIa, and FcγRIIIb. In some specific embodiments, the antibody of the present disclosure can bind to FcγRIIIA. In some embodiments, the antibody of the present disclosure can bind to FcγRIIIA and FcγRIIA, and optionally FcγRI. In one aspect, the antibody can bind to these receptors with high affinity, e.g., with a dissociation constant of less than about $10^{-7}$ M, $10^{-8}$ M, $10^{-9}$ M, or $10^{-10}$ M.

In some embodiments, the anti-CD25 antibody or the antigen-binding fragment of the present disclosure binds to the inhibitory receptor FcγRIIb with low affinity. In one aspect, the antibody binds to FcγRIIb with a dissociation constant greater than about $10^{-7}$ M, greater than about $10^{-6}$ M, or greater than about $10^{-5}$ M.

In some embodiments, the anti-CD25 antibody or the antigen-binding fragment of the present disclosure is derived from the IgG1 subclass, preferably has ADCC and/or ADCP activity. In some other embodiments, the anti-CD25 antibody of the present disclosure is derived from the IgG2 subclass.

In some embodiments, the anti-CD25 antibody or the antigen-binding fragment of the present disclosure inhibits or blocks IL-2 signaling through CD25 by less than 50%. For example, the anti-CD25 antibody or the antigen-binding fragment inhibits or blocks IL-2 signaling by less than about 40%, 35%, or 30%, preferably less than about 25%, as compared to IL-2 signaling in the absence of the antibody.

Polynucleotide and Vector

The present disclosure provides an isolated polynucleotide, which encodes the anti-CD25 antibody or the antigen-binding fragment thereof of the present disclosure. The polynucleotide may be DNA or RNA.

The present disclosure provides an expression vector comprising the polynucleotide described above, which may be a eukaryotic expression vector, a prokaryotic expression vector, or a viral vector, e.g., a plasmid, a cosmid, or a phage.

Host Cell

The present disclosure provides a host cell transformed with the expression vector described above, which may be a eukaryotic cell or a prokaryotic cell.

In some embodiments, the host cell is bacteria, yeast or a mammalian cell. In some specific embodiments, the host cell is *Escherichia coli, Pichia pastoris,* a Chinese hamster ovary (CHO) cell or a human embryonic kidney (HEK) 293 cell.

Preparation Method

The present disclosure provides a method for preparing the anti-CD25 antibody or the antigen-binding fragment thereof, which comprises: expressing the antibody or the antigen-binding fragment thereof in the above host cell, and isolating the antibody or the antigen-binding fragment thereof from the host cell.

Optionally, a purification step can also be included. For example, purification is performed using an A or G Sepharose FF column containing an adjusted buffer to wash off non-specifically bound components, and then bound antibodies are eluted by a pH gradient method, detected by SDS-PAGE, and collected. Optionally, the antibody solution can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

Methods for producing and purifying antibodies and antigen-binding fragments are well known in the prior art and can be found in, for example, "Antibodies: A Laboratory Manual", Cold Spring Harbor Press (chapters 5-8 and 15). For example, mice can be immunized with human FcRn or a fragment thereof, and the resulting antibodies can be renatured and purified, and amino acid sequencing can be performed by conventional methods. Likewise, antigen-binding fragments can be prepared by conventional methods. The engineered antibody or the antigen-binding fragment of the present disclosure can be prepared and purified by conventional methods. For example, cDNA sequences encoding the heavy and light chains can be cloned and recombined into an expression vector. Recombinant immunoglobulin expression vectors can be stably transfected into CHO cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture medium with the secreted antibody can be purified and collected by conventional techniques. The antibody can be filtered and concentrated by conventional methods. Soluble mixtures and polymers can also be removed by conventional methods, such as molecular sieves and ion exchange.

Composition

The present disclosure provides a composition, e.g., a pharmaceutical composition, which comprises a therapeutically effective amount of the anti-CD25 antibody or the antigen-binding fragment thereof described above, and a pharmaceutically acceptable excipient, diluent or carrier. In some specific embodiments, the pharmaceutical composition may contain 0.01 wt% to 99 wt% of the anti-CD25 antibody or the antigen-binding fragment thereof in a unit dose, or the anti-CD25 antibody or the antigen-binding fragment thereof contains 0.1-2000 mg, in some specific embodiments, 1-1000 mg of the pharmaceutical composition in a unit dose.

Treatment Method and Pharmaceutical Use

The present disclosure provides use of any one of or any combination of an anti-CD25 antibody or an antigen-binding fragment thereof, a pharmaceutical composition comprising the anti-CD25 antibody or the antigen-binding fragment thereof, an encoding polynucleotide for a method for diagnosing, treating or preventing a disease, and for the preparation of a medicament or a pharmaceutical composition (e.g., for treating or preventing a proliferative disorder (e.g., cancer or tumor) or delaying progression of an associated disorder).

In some embodiments, provided is a method for preventing, treating or alleviating a disorder in a subject, which comprises administering to the subject the anti-CD25 antibody or the antigen-binding fragment thereof, the pharmaceutical composition comprising the anti-CD25 antibody or the antigen-binding fragment thereof, and/or the encoding polynucleotide of the present disclosure. In some specific embodiments, the disorder in the subject is a proliferative disease, e.g., tumor or cancer. In some embodiments, the above subject has a tumor that has been formed, e.g., a solid tumor. In some embodiments, provided is a method for reducing the number of intratumoral or tumor-infiltrating Treg cells in a subject; in some embodiments, provided is a method for eliminating or inhibiting the activity of intratumoral or tumor-infiltrating Treg cells in a subject, both of which comprise administering to the subject the anti-CD25 antibody or the antigen-binding fragment thereof, the pharmaceutical composition comprising the anti-CD25 antibody or the antigen-binding fragment thereof, and/or the encoding polynucleotide of the present disclosure.

In some embodiments, provided is a method for increasing the ratio of Teffs/Tregs in a tumor in a subject, which comprises administering to the subject the anti-CD25 antibody or the antigen-binding fragment thereof, the pharmaceutical composition comprising the anti-CD25 antibody or the antigen-binding fragment thereof, and/or the encoding polynucleotide of the present disclosure. In some specific embodiments, the ratio of effector T cells to regulatory T cells (Teffs/Tregs) in a tumor is increased to greater than 5, 10, 15, 20, 40, or 80.

In some embodiments, provided is a method for enhancing CDC, ADCC and/or ADCP against a tumor cell in a subject, which comprises administering to the subject the anti-CD25 antibody or the antigen-binding fragment thereof, the pharmaceutical composition comprising the anti-CD25 antibody or the antigen-binding fragment thereof, and/or the encoding polynucleotide of the present disclosure. In some specific embodiments, the ADCC and/or ADCP effect against a tumor cell in a subject is enhanced. In some more specific embodiments, the ADCC effect against a tumor cell in a subject is enhanced.

In some embodiments, provided is pharmaceutical use of the anti-CD25 antibody or the antigen-binding fragment thereof, the pharmaceutical composition comprising the anti-CD25 antibody or the antigen-binding fragment thereof, and/or the encoding polynucleotide of the present disclosure for the preparation of a medicament for preventing, treating or alleviating a disorder in a subject, for the preparation of a medicament for reducing the number of intratumoral or tumor-infiltrating Treg cells in a subject, for the preparation of a medicament for eliminating or inhibiting the activity of intratumoral or tumor-infiltrating Treg cells in a subject, for the preparation of a medicament for increasing the ratio of Teffs/Tregs in a tumor in a subject, and for the preparation of a medicament for enhancing CDC, ADCC and/or ADCP against a tumor cell in a subject.

In some specific embodiments, the disorder in the above subject is a proliferative disorder (e.g., cancer or tumor) or the subject has a proliferative disorder (e.g., cancer or tumor). The tumor includes, but is not limited to, carcinoma, lymphoma, leukemia, blastoma, and sarcoma. More specific examples of such cancers include squamous cell carcinoma, myeloma, small-cell lung cancer, non-small cell lung cancer, neuroglioma, hepatocellular carcinoma (HCC), Hodgkin's lymphoma, non-Hodgkin's lymphoma, acute myeloid leukemia (AML), multiple myelomas, gastrointestinal cancer, kidney cancer, ovarian cancer, liver cancer, lymphoblastic leukemia, lymphocytic leukemia, colorectal cancer, endometrial cancer, renal cancer, prostate cancer, thyroid cancer, melanoma, chondrosarcoma, neuroblastoma, pancreatic cancer, glioblastoma multiforme, cervical cancer, brain cancer, gastric cancer, bladder cancer, hepatoma, breast cancer, colon cancer, and head and neck cancer.

In some specific embodiments, the cancer or tumor may be a solid tumor, including but not limited to, sarcoma (including cancers arising from transformed cells of mesenchymal origin in tissue (e.g., cancellous bone, cartilage, fat, muscle, blood vessels, hematopoietic cells, or fibrous connective tissue)), carcinoma (including tumors arising from epithelial cells), mesothelioma, neuroblastoma, retinoblastoma, etc. Cancers involving solid tumors include, but are not limited to, brain cancer, lung cancer, stomach cancer, duodenal cancer, esophageal cancer, breast cancer, colon and rectal cancer, renal cancer, bladder cancer, kidney cancer, pancreatic cancer, prostate cancer, ovarian cancer, melanoma, oral cancer, sarcoma, eye cancer, thyroid cancer, urethral cancer, vaginal cancer, neck cancer, lymphoma, etc.

In some specific embodiments, the cancer is related to tumors expressing CD25, including but not limited to, lymphomas such as Hodgkin's lymphoma and lymphocytic leukemia (e.g., chronic lymphocytic leukemia (CLL)).

Detection Use

The present disclosure provides detection use of the anti-CD25 antibody or the antigen-binding fragment thereof.

The present disclosure provides a reagent for detecting CD25, which comprises the anti-CD25 antibody or the antigen-binding fragment thereof. The present disclosure also provides a method, system, or device for detecting CD25 *in vivo* or *in vitro,* which comprises using the anti-CD25 antibody or the antigen-binding fragment thereof.

In some embodiments, the *in vitro* detection method, system or device may, for example, comprise (1) making a sample in contact with an anti-CD25 antibody or an antigen-binding fragment thereof; (2) detecting a complex formed between the anti-CD25 antibody or the antigen-binding fragment thereof and the sample; and/or (3) making a reference sample (e.g., a control sample) in contact with the antibody; and (4) determining the extent of complex formation between the antibody and the sample by comparison with a reference sample. A change (e.g., a statistically significant change) in complex formation in the sample or subject as compared to a control sample or subject indicates the presence of CD25 in the sample.

In some embodiments, the CD25-binding protein or the anti-CD25 antibody of the present disclosure may be labeled with a fluorophore and a chromophore for detection purposes. In some embodiments, also provided is a kit, which comprises a CD25-binding protein or an anti-CD25 antibody, and may also comprise instructions for diagnostic use. The kit may also comprise at least one additional reagent, such as a label or an additional diagnostic agent. For *in vivo* use, the antibody may be formulated into a pharmaceutical composition.

The anti-CD25 antibody or the antigen-binding fragment thereof provided by the embodiments of the present disclosure features high specificity, high affinity and low immunogenicity. Meanwhile, the antibody of the present disclosure has the effects of well inhibiting Treg, not affecting Teff, enhancing ADCC, ADCP and/or CDC in a subject and inhibiting tumorigenesis and development.

**BRIEF DESCRIPTION OF THE DRAWINGS**

FIGs. 1A and 1B: identification of the activity of recombinant proteins by ELISA. FIG. 1A shows the assay results of the activity of the recombinant human CD25 protein, and
FIG. 1B shows the assay results of the activity of the recombinant monkey and mouse CD25 proteins.
FIG. 2: assay on the cell strain 2F8 stably expressing human CD25 protein by FACS, in which the white peak is CD25 positive peak, the gray peak is control peak, and the primary antibody is human IgG1 negative control antibody.
FIGs. 3A to 3C: assay on the antibody function by FACS. FIG. 3A shows the assay results of binding of antibodies DAC, 7G7B6 and Tab06 to CHO-K1 cells by FACS, FIG. 3B shows the assay results of binding of antibodies DAC, 7G7B6 and Tab06 to CHO-K1-CD25 cells stably expressing human CD25 protein by FACS, and FIG. 3C shows the assay results of binding of antibodies DAC, 7G7B6 and Tab06 to Su-DHL-1 by FACS, in which mouse IgG or human IgG isotype is used as a negative control.
FIGs. 4A to 4C: assay on the binding of antibodies to CD25 by ELISA. FIG. 4A shows the binding results of 7G7B6 and Tab06 to recombinant human CD25 protein, FIG. 4B shows the binding results of 7G7B6 and Tab06 to recombinant monkey CD25 protein, and FIG. 4C shows the binding results of 7D4 to recombinant mouse CD25 protein, in which the negative controls used are mouse IgG and human IgG.
FIGs. 5A to 5C: assay on the binding of chimeric anti-CD25 antibodies to SU-DHL-1 cells by FACS. FIG. 5A shows the assay results of cAb001, cAb002, cAb004 and cAb006,
FIG. 5B shows the assay results of cAb028, cAb029, and cAb037, and FIG. 5C shows the assay results of cAb042 and cAb046, in which the positive controls used are Tab06 and DAC, and the negative control is human IgG (i.e., hIgG).
FIGs. 6A to 6G: assay on the binding of chimeric anti-CD25 antibodies to the CD25 antigen on Treg cells and activated CD4$^+$ and CD8$^+$ effector T cells by FACS. FIG. 6A shows the result for cAb006, FIG. 6B shows the result for cAb037, FIG. 6C shows the result for cAb042, FIG. 6D shows the result for cAb046, FIG. 6E shows the result for a positive control 7G7B6, FIG. 6F shows the result for a positive control DAC, and FIG. 6G shows the result for a positive control Tab06.
FIGs. 7A to 7B: assay on the effect of chimeric anti-CD25 antibodies on the binding ability of IL-2 to the receptor CD25 by FACS. FIG. 7A is a graph showing the assay results of cAb001, cAb002, cAb028 and cAb029, FIG. 7B is a graph showing the assay results of cAb006, cAb037, cAb042, and cAb046, in which the anti-CD25 antibody control used for inhibiting the binding of IL-2 to CD25 is DAC, the anti-CD25 antibody control not inhibiting the binding of IL-2 to CD25 is Tab06, and the negative control is human IgG (i.e., hIgG-1).
FIG. 8: assay on the binding of humanized anti-CD25 antibodies to SU-DHL-1 cells by FACS.
FIG. 9: assay on the effect of humanized anti-CD25 antibodies on the human peripheral blood T lymphocyte pStat5 signaling pathway by FACS.
FIG. 10: assay on the ADCC effect on SU-DHL-1 cells mediated by humanized anti-CD25 antibodies by determining the fluorescent activity of an ADCC reporter gene in the cells.
FIGs. 11A and 11B: results of the antitumor activity against MC38 xenograft tumor in hCD25 mice mediated by humanized anti-CD25 antibody, in which FIG. 11A is a tumor inhibition effect graph, and FIG. 11B is a corresponding mouse body weight graph.
FIGs. 12A and 12B: analysis results of intratumoral lymphocytes of MC38 xenograft tumor of hCD25 mice mediated by the humanized anti-CD25 antibodies, in which FIG. 12A shows the results of killing of Tregs by the antibodies and FIG. 12B shows the up-regulation of CD3 by the antibodies.

**DETAILED DESCRIPTION**

Detailed Description of the Invention

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The three-letter and single-letter codes for amino acids used in the present disclosure are described as in J. Biol. Chem, 243, p3558 (1968).

The term "CD25", "CD25 protein" or "CD25 polypeptide" may optionally include any such protein or a variant, a conjugate or a fragment thereof, including (but not limited to) known or wild-type CD25 described herein, as well as any naturally occurring splice variant, amino acid variant or isoform. The intact human CD25 sequence can be found under Uniprot accession No. P01589, whose amino acids 22 to 240 correspond to the extracellular domain of mature human CD25.

The term "binding to CD25" refers to the ability to interact with CD25 or an epitope thereof, wherein the CD25 or the epitope thereof may be derived from humans. The term "antigen-binding site" refers to a discontinuous three-dimensional spatial site on an antigen that is recognized by the antibody or the antigen-binding fragment of the present disclosure.

The term "antibody" refers to an immunoglobulin, which is of a tetrapeptide chain structure formed by connection between two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA and IgE, with their corresponding heavy chains being $\mu$ chain, $\delta$ chain, $\gamma$ chain, $\alpha$ chain and $\varepsilon$ chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into $\kappa$ or $\lambda$ chains by the differences in the constant regions. Each of the five classes of Ig may have a $\kappa$ chain or $\lambda$ chain. In the heavy and light chains of antibody, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable region (VHs) consists of 3 CDR regions and 4 FR regions arranged from the amino terminus to the carboxyl terminus in the following order: FR1, CDR1, FR2, CDR2, FR3, CDR3, and FR4. The 3 CDR regions of the light chain refer to LCDR1, LCDR2, and LCDR3; and the 3 CDR regions of the heavy chain refer to HCDR1, HCDR2, and HCDR3. In some embodiments, the antibody of the present disclosure specifically or substantially specifically binds to CD25.

The term "antibody binding to CD25" refers to an antibody that is capable of binding to the CD25 subunit of the IL-2 receptor. This subunit is also known as the $\alpha$ subunit of the IL-2 receptor. Such antibodies are also referred to herein as "anti-CD25 antibodies". The CDR amino acid residues of the VL and VH regions of the antibody or the antigen-binding fragment of the present disclosure correspond with known Kabat numbering scheme in terms of number and positions. EU numbering in Kabat is also generally used for constant domains and/or Fc domains.

For determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any of a variety of techniques known to those skilled in the art, such as X-ray crystallography. A variety of analysis methods can be used to identify CDRs, including but not limited to Kabat numbering scheme, Chothia numbering scheme, AbM numbering scheme, IMGT numbering scheme, contact definition, and conformational definition. The Kabat numbering scheme is a standard for numbering residues in antibodies and is generally used to identify CDRs (see, e.g., Johnson & Wu, 2000, Nucleic Acids Res., 28: 214-8). The Chothia numbering scheme is similar to the Kabat numbering scheme, except that it takes into account the position of certain structural loop regions. (see, e.g., Chothia et al., 1986, J. Mol. Biol., 196: 901-17; Chothia et al., 1989, Nature, 342: 877-83). The AbM numbering scheme adopts a computer program integration suite for modeling antibody structures manufactured by Oxford Molecular Group (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3: 194-198). The contact definition is based on the analysis of the available complex crystal structures (see, e.g., MacCallum et al., 1996, J. Mol. Biol., 5: 732-45). In the conformational definition, the positions of the CDRs can be identified as residues that contribute enthalpy to the antigen binding (see, e.g., Makabe et al., 2008, Journal of Biological Chemistry, 283: 1156-1166). In addition, other CDR boundary definitions may not strictly follow one of the above methods, but still overlap with at least a portion of the Kabat CDRs, although they may be shortened or lengthened based on predictions or experimental results that a particular residue or a particular group of residues do not significantly affect the antigen binding. As used herein, CDRs may refer to CDRs defined by any method known in the art, including combinations of methods. In the methods used herein, CDRs defined according to any of those methods may be used. The CDRs are defined using the Kabat numbering scheme in the examples of the disclosure, but one skilled in the art will understand that the CDRs may also be redefined according to any of the Chothia, extended, AbM, IMGT, contact, and/or conformational definitions.

The Fc region of IgG antibodies interacts with several cellular Fcγ receptors (FcγRs) to stimulate and regulate downstream effector mechanisms. There are five activating receptors, namely FcγRI (CD64), FcγRIIa (CD32a), FcγRIIc (CD32c), FcγRIIIa (CD16a) and FcγRIIIb (CD16b), and one inhibitory receptor FcγRIIb (CD32b). Communication of IgG antibodies with the immune system is controlled and mediated by FcγR, which transmits information sensed and collected by the antibodies to the immune system, thereby providing an association between the innate and adaptive immune systems, particularly in the context of biotherapy (Hayes J et al, 2016. J Inflamm Res 9: 209-219). The IgG subclasses differ in their ability to bind to FcγR, and this differential binding determines their ability to trigger a range of functional responses. For example, in humans, FcγRIIIa is the main receptor involved in antibody-dependent cell-mediated cytotoxicity (ADCC) activation, and IgG1, immediately followed by IgG3, shows the highest affinity for this receptor, which reflects their ability to effectively induce ADCC. IgG2 has weak binding to this receptor, but anti-CD25 antibodies of human IgG2 isotype have been found to be effective in depleting Tregs as well.

The term "regulatory T cells", "Tregs" or "Treg cells" refers to the $CD4^+$ T lymphocyte lineage that is specialized in the control of autoimmunity, allergy and infection. Typically, they regulate the activity of T cell population, but they may also affect certain innate immune system cell types. Tregs can be identified by expression of the biomarkers CD4, CD25 and Foxp3. Naturally occurring Treg cells typically account for about 5% to 10% of peripheral $CD4^+$ T lymphocytes. However, in the tumor microenvironment (i.e., tumor-infiltrating Treg cells), they may account for 20% to 30% of the total $CD4^+$ T lymphocyte population. Activated human Treg cells can directly kill target cells such as Teffs and APCs (antigen presenting cells) through either a perforin or granzyme B-dependent pathway; cytotoxic T lymphocyte-associated antigen 4 ($CTLA4^+$) Treg cells induce the expression of indoleamine 2,3-dioxygenase (IDO) by APC, which in turn inhibits T cell activation by reducing tryptophan; Treg cells can release interleukin-10 (IL-10) and transforming growth factor (TGFβ) *in vivo,* thereby directly inhibiting T cell activation and inhibiting APC function by inhibiting the expression of MHC molecules, CD80, CD86 and IL-12. Treg cells can also suppress immunity by expressing high levels of CTLA4, which can bind to CD80 and CD86 on antigen presenting cells and prevent the correct activation of effector T cells.

The term "immune effector cell" herein refers to an immune cell involved in the effector phase of an immune response. Exemplary immune cells include myeloid or lymphoid cells, such as lymphocytes (e.g., B cells and T cells including cytolytic T Cells (CTLs)), killer cells, natural killer cells, macrophages, monocytes, eosinophils, neutrophils, polymorphonuclear cells, granulocytes, mast cells, and basophils.

The terms "not inhibiting", "not blocking", "non-blocking", "non-II,-2-blocking", "no blocking", and the like (with respect to not blocking or not inhibiting the binding of IL-2 to CD25, e.g., "not inhibiting IL-2 binding to CD25", in the presence of an anti-CD25 antibody) herein include the case where the anti-CD25 antibody does not block or does not inhibit IL-2 signaling through CD25, and in particular does not inhibit interleukin-2 signaling in cells expressing CD25. That is, the anti-CD25 antibody of the present disclosure inhibits IL-2 signaling through CD25 by less than 50%, as compared to IL-2 signaling in the absence of the antibody. Preferably, the anti-CD25 antibody inhibits IL-2 signaling by less than about 40%, 35% or 30%, preferably less than about 25%, as compared to IL-2 signaling in the absence of the antibody.

The term "antibody-dependent cell-mediated cytotoxicity" (ADCC) refers to a cell-mediated response in which nonspecific cytotoxic cells expressing Fc receptors (FcRs) (e.g., natural killer (NK) cells, neutrophils, and macrophages) recognize bound antibody on a target cell, resulting in lysis of the target cell. The term "antibody-dependent cell-mediated phagocytosis" (ADCP) refers to a cell-mediated response in which phagocytic cells expressing Fc receptors (FcRs) (e.g., macrophages) recognize bound antibody on a target cell, resulting in phagocytosis of the target cell. The term "complement dependent cytotoxicity" (CDC) refers to the lysis of cells expressing the antigen by antibodies in the presence of complement. CDC, ADCC and ADCP can be determined by assay methods known and available in the art (Clynes et al (1998) Proc Natl Acad Sci USA 95,652-6), and the constant region of an antibody is important in the ability of the antibody to fix complement and mediate cell-dependent cytotoxicity and phagocytosis. Therefore, the isotype of an antibody may be selected based on whether the antibody needs to mediate ADCC and ADCP.

ADCC can be increased by methods that eliminate the fucose moiety from the antibody glycan, for example by producing antibodies in the YB2/0 cell line, or by introducing specific mutations (e.g., S298A/E333A/K334A, S239D/I332E/A330L, G236A/S239D/A330L/I332E) on the Fc portion of human IgG1 (Lazar et al. (2006) Proc Natl Acad Sci USA 103, 2005-2010; Smith et al.(2012)Proc Natl Acad Sci USA 109,6181-6186). ADCP can also be increased by introducing specific mutations in the Fc portion of human IgG1 (Richards et al (2008) Mol Cancer Ther 7, 2517-2527).

The term "murine antibody" in the present disclosure refers to a monoclonal antibody against human CD25 or an epitope thereof prepared according to the knowledge and skill in the art. During the preparation, a test subject is injected with a CD25 antigen, and then hybridoma of antibodies expressing the desired sequence or functional properties is isolated. In a specific embodiment of the present disclosure, the murine anti-human CD25 antibody or the antigen-binding fragment thereof may further comprise a light chain constant region of a murine κ or λ chain or a variant thereof, or further comprise a heavy chain constant region of a murine IgG1, IgG2, IgG3 or IgG4 or a variant thereof.

The term "chimeric antibody" refers to an antibody obtained by fusing a variable region of a murine antibody and a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, linking the mouse variable region gene and the human constant region gene into a chimeric gene, inserting the chimeric gene into a human vector, and finally expressing chimeric antibody molecules in a eukaryotic industrial system or prokaryotic industrial system. The constant region of the human antibody may be selected from the group consisting of the heavy chain constant regions of human IgG1, IgG2, IgG3 and IgG4 or variants thereof, preferably comprising human IgG2 or IgG4 heavy chain constant regions, or IgG1 mutated at amino acids without ADCC (antibody-dependent cell-mediated cytotoxicity) toxicity.

The term "humanized antibody", also known as a CDR-grafted antibody, refers to an antibody produced by grafting murine CDR sequences into the framework of variable regions of a human antibody. Such antibody can overcome the strong immune response induced by the chimeric antibody because of carrying a large amount of mouse protein components. To avoid the decrease in activity caused by the decrease in immunogenicity, the variable regions of a human antibody can be subjected to minimum reverse mutation to maintain activity. The antibody of the present disclosure may be an affinity-matured humanized antibody, and the CDRs of the parent sequences (including all sequences) of the affinity-matured antibody are at least 80% identical, e.g., 90% identical. An affinity-matured antibody is an antibody with one or more altered amino acids in one or more CDRs, which results in an improvement of the affinity of an antibody for CD25 as compared to a parent antibody without the altered amino acids.

The term "human antibody" includes antibodies having variable and constant regions derived from human germline immunoglobulin sequences. The human antibody of the present disclosure may include amino acid residues not encoded by human germline immunoglobulin sequences (e.g., mutations introduced by random or site-specific mutagenesis *in vitro* or by somatic mutations *in vivo*). However, "human antibody" does not include antibodies in which CDR sequences derived from the germline of another mammalian species (such as mice) have been grafted into a human framework sequence (i.e., "humanized antibodies").

The term "antigen-binding fragment" includes a single-chain antibody (i.e., full-length heavy and light chains); Fab, a modified Fab, Fab', a modified Fab', F(ab')$_2$, Fv, Fab-Fv, Fab-dsFv, a single domain antibody (e.g., VH or VL or VHH), scFv, a bivalent or trivalent or tetravalent antibody, Bis-scFv, a diabody, a tribody, a triabody, a tetrabody and an epitope-binding fragment of any of the above (see, e.g., Holliger and Hudson, 2005, Nature Biotech. 23 (9): 1126-1136; Adair and Lawson, 2005, Drug Design Reviews-Online 2 (3), 209-217). Methods for producing and preparing such antibody fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also include Fab and Fab' fragments described in WO2005/003169, WO2005/003170 and WO2005/003171. Multivalent antibodies may comprise multiple specificities (e.g., bispecificites) or may be monospecific (see, e.g., WO92/22583 and WO05/113605), and an example of the latter is Tri-Fab (or TFM) described in WO92/22583. In some specific embodiments, the anti-CD25 antibody of the present disclosure encompasses bispecific and multispecific antibodies.

The term "epitope" refers to a site on an antigen to which an immunoglobulin or an antibody specifically binds. An epitope may be formed from contiguous amino acids, or non-contiguous amino acids juxtaposed by tertiary folding of the protein. An epitope formed from contiguous amino acids are generally retained after exposure to a denaturing solvent, while an epitope formed by tertiary folding are generally lost after a denaturing solvent treatment. An epitope generally comprise, for example, at least 3-15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described herein, such as X-ray crystallography and two-dimensional nuclear magnetic resonance.

The term "specific binding" or "selective binding" refers to binding of an antibody to an epitope on a predetermined antigen. Typically, an antibody binds to a predetermined antigen or epitope thereof with an equilibrium dissociation constant (KD) of about less than $10^{-7}$ M or even less and with an affinity that is at least twice as high as its affinity for binding to a non-specific antigen other than the predetermined antigen (or epitope thereof) or a closely related antigen (e.g., BSA, etc.), when determined by surface plasmon resonance (SPR) techniques in an instrument using recombinant human CD25 or an epitope thereof as the analyte and an antibody as the ligand. The term "antigen-recognizing antibody" is used interchangeably herein with "specifically bound antibody". The term "binding affinity" refers to the apparent association constant or Ka. Ka is the reciprocal of the dissociation constant (Kd). For example, a binding protein may have a binding affinity of at least $10^{-5}$ M, $10^{-6}$ M, $10^{-7}$ M, $10^{-8}$ M, $10^{-9}$ M, $10^{-10}$ M, and $10^{-11}$ M for a particular target molecule. Higher affinity binding of the binding partner to the first target relative to the second target may be indicated by a higher value of Ka (or Kd) for binding to the first target than Ka (or Kd of a smaller value) for binding to the second target. In these cases, the binding protein is specific for the first target (e.g., a protein in the first conformation or analog thereof) relative to the second target (e.g., the same protein in the second conformation or analog thereof; or a second protein). The difference in binding affinity (e.g., for specificity or other comparisons) is at least 1.5 fold, 2 fold, 3 fold, 4 fold, 5 fold, 10 fold, 15 fold, 20 fold, 50 fold, 70 fold, 80 fold, 100 fold, 500 fold, 1000 fold, or 105 fold.

The term "conservative substitution" refers to a substitution to another amino acid residue having similar properties to the original amino acid residue. For example, lysine, arginine and histidine have similar properties in that they have basic side chains, and aspartic acid and glutamic acid have similar properties in that they have acidic side chains. In addition, glycine, asparagine, glutamine, serine, threonine, tyrosine, cysteine and tryptophan have similar properties in that they have uncharged polar side chains, and alanine, valine, leucine, threonine, isoleucine, proline, phenylalanine and methionine have similar properties in that they have nonpolar side chains. In addition, tyrosine, phenylalanine, tryptophan and histidine have similar properties in that they have aromatic side chains. Thus, it will be apparent to those skilled in the art that even when an amino acid residue in a group exhibiting similar properties as described above is substituted, it will not exhibit a particular change in properties.

The term "sequence homology" or "sequence identity" refers to sequence similarity between two polynucleotide sequences or between two polypeptides. When positions in both compared sequences are occupied by the same base or amino acid monomer subunit, e.g., if each position of two DNA molecules is occupied by adenine, then the molecules are homologous at that position. The homology percentage between two sequences is a function of the number of matching or homologous positions shared by the two sequences divided by the number of positions compared $\times$ 100%. For example, if 6 out of 10 positions are matched or homologous when two sequences are optimally aligned, the two sequences are 60% homologous. Generally, when two sequences are aligned, comparison is performed to obtain the maximum homology percentage.

The term "cross-reactivity" refers to the ability of the antibody of the present disclosure to bind to CD25 from different species. For example, the antibody of the present disclosure that binds to human CD25 may also bind to CD25 from another species. Cross-reactivity is determined by detecting specific reactivity with purified antigen in binding assays (e.g., SPR and ELISA) or binding or functional interactions with cells physiologically expressing CD25. Methods for determining cross-reactivity include standard binding assays as described herein, for example, surface plasmon resonance analysis or flow cytometry.

The terms "inhibition" and "blocking" are used interchangeably and encompass both partial and complete inhibition/blocking. Inhibition/blocking of CD25 preferably reduces or alters the normal level or type of activity that occurs when CD25 binding occurs without inhibition or blocking. Inhibition and blocking are also intended to include any measurable decrease in CD25 binding affinity when contacted with an anti-CD25 antibody compared to CD25 not contacted with an anti-CD25 antibody.

The term "inhibition of growth" (e.g., involving cells) is intended to include any measurable reduction in cell growth.

Methods for producing and purifying antibodies and antigen-binding fragments are well known in the prior art and can be found in, for example, "Antibodies: A Laboratory Manual", Cold Spring Harbor Press (chapters 5-8 and 15). For example, mice can be immunized with human CD25 or a fragment thereof, and the resulting antibodies can be renatured and purified, and amino acid sequencing can be performed by conventional methods. Likewise, antigen-binding fragments can be prepared by conventional methods. The antibody or the antigen-binding fragment described in the present invention is genetically engineered to contain one or more additional human FRs in the non-human-derived CDRs. Human FR germline sequences can be obtained from the ImMunoGeneTics (IMGT) website http://imgt.cines.fr or from the Immunoglobulin Journal, 2001ISBN012441351.

The antibody of the present disclosure may be polyclonal, monoclonal, xenogenic, allogeneic, syngeneic, or modified forms thereof, with the monoclonal antibody being particularly useful in various embodiments. Generally, the antibody of the present disclosure is a recombinant antibody. The "recombinant" used herein generally refers to such products as a cell, a nucleic acid, a protein or a vector, and indicates that the cell, the nucleic acid, the protein or the vector has been modified by the introduction of a heterologous nucleic acid or protein or the alteration of a native nucleic acid or protein, or that the cell is derived from a cell modified in this way. For example, recombinant cells express genes that are not found within the native (non-recombinant) cellular form or express native genes that are abnormally expressed, under expressed or not expressed at all.

The term "monoclonal antibody" or "mAb" refers to an antibody derived from a single clonal cell strain, which is not limited to eukaryotic, prokaryotic, or phage clonal cell strains. The monoclonal antibody or the antigen-binding fragment can be obtained by, for example, hybridoma technology, recombinant technology, phage display technology, synthetic technology (e.g., CDR-grafting), or other technologies known in the art.

The antibody or the antigen-binding fragment of the present disclosure can be prepared and purified by conventional methods. For example, cDNA sequences encoding the heavy and light chains can be cloned and recombined into an expression vector. Recombinant immunoglobulin expression vectors can be stably transfected into CHO cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture medium with the secreted antibody can be purified and collected by conventional techniques. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Publication No. WO03/48731. Therefore, an antibody and an antigen-binding fragment thereof that competes with the antibody molecule of the present disclosure for binding to the same epitope on CD25 can be obtained by conventional techniques known to those skilled in the art. The method for detecting competitive binding of antibodies in WO2018167104 is incorporated herein in its entirety. "Giving", "administering" and "treating", when applied to animals, humans, experimental subjects, cells, tissues, organs or biological fluid, refer to contact of an exogenous drug, a therapeutic agent, a diagnostic agent or a composition with the animals, humans, subjects, cells, tissues, organs or biological fluid. "Giving", "administering" and "treating" can refer to, for example, therapeutic, pharmacokinetic, diagnostic, research, and experimental methods. The treatment of the cells comprises contacting the reagent with the cells and contacting the reagent with fluid, where the fluid is in contact with the cells. "Giving", "administering" and "treating" also refer to treating, e.g., a cell, by a reagent, diagnosis, a binding composition, or by another cell *in vitro* and *ex vivo*. "Treating", when applied to humans, veterinary or research subjects, refers to therapeutic treatment, preventive or prophylactic measures, and research and diagnostic applications. "Treating" or "treatment" refers to administering a therapeutic agent, such as a composition comprising any of the antibodies or the antigen-binding fragments thereof of the present disclosure or conjugates thereof, either internally or externally to a subject who has had, is suspected of having, or is predisposed to having one or more diseases or symptoms thereof on which the therapeutic agent is known to have a therapeutic effect. Typically, the therapeutic agent is administered in an amount effective to alleviate one or more symptoms of the disease in the subject or population being treated, whether by inducing regression of such symptoms or inhibiting the development of such symptoms into any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating symptoms of a disease of interest in a certain subject, they shall alleviate the symptoms of the disease of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

The term "treating cancer" used herein includes (a) inhibiting cancer, i.e., arresting its development, including but not limited to, blocking or delaying the progression of cancer, blocking or delaying metastasis of cancer; and/or (b) alleviating cancer, i.e., causing regression of the cancer, including but not limited to, relieving or alleviating one or more symptoms associated with the cancer, relieving or alleviating metastatic cancer, and/or reducing or eliminating the tumor.

The term "prevention" herein refers to delaying or preventing the onset of the symptoms of cancer. The prevention may be absolute (and thus no disease occurs) or effective only in certain individuals or for a limited period of time.

"Effective amount" comprises an amount sufficient to ameliorate or prevent a symptom or sign of a medical condition. An effective amount also refers to an amount sufficient to allow or facilitate diagnosis. The effective amount for a particular subject or veterinary subject may vary depending on the factors such as the condition to be treated, the general health of the subject, the method and route and dosage of administration, and the severity of side effects. An effective amount can be the maximum dose or administration regimen to avoid significant side effects or toxic effects.

"Cell", "cell line" and "cell culture" are used interchangeably, and all such designations include their progenies. It should also be understood that all progenies may not be precisely identical in DNA content due to deliberate or unintentional mutations. Mutant progeny with the same function or biological activity as screened in the original transformed cells is included.

The term "optional" or "optionally" means that the event or circumstance subsequently described may, but not necessarily, occur, and that the description includes instances where the event or circumstance occurs or does not occur. For example, "optionally comprising 1-3 antibody heavy chain variable regions" means that the antibody heavy chain variable region of a particular sequence may, but not necessarily, be present.

"Pharmaceutical composition" refers to a mixture containing one or more of the antibodies and the antigen-binding fragments described herein or a physiologically/pharmaceutically acceptable salt or pro-drug thereof, and other chemical components such as physiologically/pharmaceutically acceptable carriers and excipients. The purpose of the pharmaceutical composition is to promote the administration to an organism, which facilitates the absorption of the active ingredient, thereby exerting biological activities.

"Tumor" is applicable to a subject diagnosed with or suspected of having a tumor, and "cancer" refers to a malignant or potentially malignant neoplasm or mass of tissue of any size, and includes both primary tumors and secondary neoplasms. "Cancer", "malignancy", "neoplasm", "tumor" and "cancer" are also used interchangeably herein to refer to tumors and tumor cells that exhibit relatively abnormal, uncontrolled and/or autonomous growth, such that they exhibit an abnormal growth phenotype characterized by a significant loss of control over cell proliferation. Typically, cells for detection or treatment include precancerous (e.g., benign), malignant, pre-metastatic, metastatic and non-metastatic cells. "Solid tumor" is an abnormal growth or mass of tissue that generally does not contain cysts or fluid areas, particularly tumors and/or metastases (wherever located) other than leukemia or non-solid lymphoma. The solid tumor may be benign or malignant. Different types of solid tumors are named for the type of cells from which they are formed and/or the tissue or organ in which they are located. Examples of solid tumors include, but are not limited to, sarcoma (including cancers arising from transformed cells of mesenchymal origin in tissue (e.g., cancellous bone, cartilage, fat, muscle, blood vessels, hematopoietic cells, or fibrous connective tissue)), carcinoma (including tumors arising from epithelial cells), melanoma, lymphoma, mesothelioma, neuroblastoma, and retinoblastoma.

The articles "a" and "an" used herein refer to one or to more than one (i.e., at least one) grammatical objects of the article, unless otherwise specified. For example, "an element" refers to one or more than one element.

## DETAILED DESCRIPTION

The present disclosure is further described below with reference to examples, which, however, are not intended to limit the scope of the present disclosure.

Experimental procedures without specific conditions indicated in the examples or test examples are generally conducted according to conventional conditions, or according to conditions recommended by the manufacturer of the starting materials or commercial products, see Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

### Example 1. Assay on the Activity of Recombinant CD25 Proteins

The recombinant human CD25-Fc fusion protein (purchased from ACRO Biosystems) was prepared by fusing a human IgG1 Fc tag protein to the C-terminus of a human CD25 protein (GenBank, accession number: NP-000408.1). The recombinant human CD25-His protein (purchased from Sino Biological Inc.) was prepared by fusing a poly-histidine tag to the C-terminus of a human CD25 protein (GenBank, accession number: NP-000408.1) Met1-Cys213. The recombinant monkey CD25-His protein (purchased from Sino Biological Inc.) was prepared by fusing a His tag to the C-terminus of a monkey CD25 protein (GenBank, accession number: NP-001270633.1) Met1-Arg213. The recombinant mouse CD25-His protein (purchased from Sino Biological Inc.) was prepared by fusing a His tag to the C-terminus of a mouse CD25 protein (GenBank, accession number: NP-032393.3) Met1-Lys236. The anti-CD25 antibodies DACs (Efalizumab and daclizumab) were used to perform a series of quality control tests, such as activity verification, on the purchased recombinant proteins.

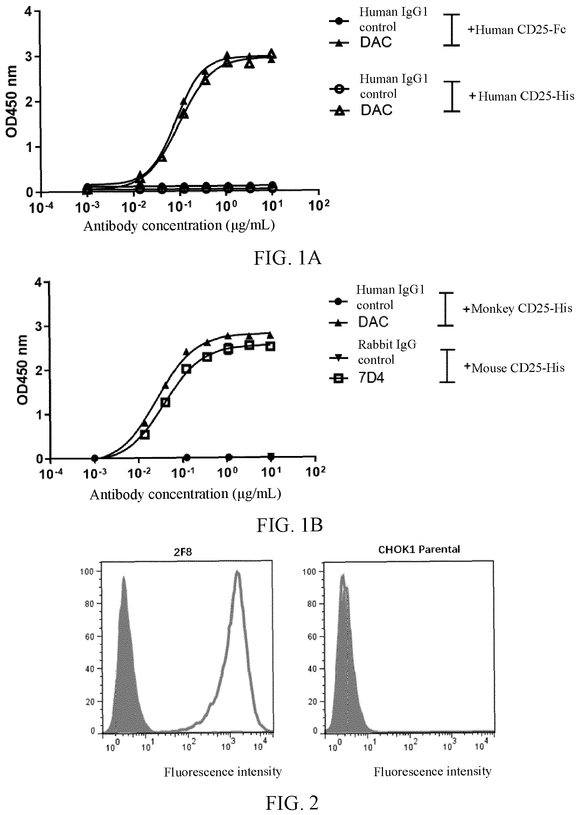

The activity of the recombinant proteins was verified by ELISA (enzyme-linked immunosorbent assay). The specific procedures were as follows: the recombinant human CD25-Fc fusion protein, the human CD25-His protein, the monkey CD25-His protein and the mouse CD25-His protein each were diluted to 1 $\mu$g/mL with PBS, and the diluted proteins were added into an ELISA microplate at 100 $\mu$L/well, and incubated at 4 °C overnight; an ELISA blocking solution (PBS buffer containing 1% BSA (w/v), pH 7.4) was added, the plate was blocked at 37 °C for 2 h, test antibodies DAC and 7D4 (anti-mouse CD25 antibody) diluted in a gradient were sequentially added, and the plate was incubated at 37 °C for 1 h and washed for 2-3 times with a washing solution; a horseradish peroxidase-labeled (HRP) secondary antibody was added, and the plate was incubated at 37 °C for 1 h and washed 2-3 times with a washing solution. 100 $\mu$L of TMB substrate was added into each well, and after incubation for 15 min at room temperature, 50 $\mu$L of stop solution (2M HCl) was added into each well. OD450nm values were read using an ELISA plate reader (SpectraMax M5e). The results are shown in FIGs. 1A and 1B.

The results show that the two recombinant human CD25 proteins and the recombinant monkey CD25-His protein all can bind to DAC, and the detection signals are changed along with the change of the concentration of test antibodies; the recombinant mouse CD25-His protein cannot bind to DAC, but can bind to 7D4, suggesting that the above recombinant CD25 proteins are all active.

### Example 2. Construction of Stable Cell Strain Expressing Recombinant Human CD25 Protein

After CHO-K1 cells were transfected with a plasmid encoding a recombinant human CD25 protein, cell strains stably expressing a recombinant human CD25 protein were obtained by screening. The nucleotide sequence encoding human CD25 was cloned into a pCDNA3.4 vector (purchased from Invitrogen), and plasmids were prepared. CHO-K1 cell lines (all purchased from Invitrogen) were transfected with Lipofectamine 2000, selectively cultured for 2 weeks in a CD-CHO medium containing G418, subcloned in a 96-well culture plate by a limiting dilution method, and cultured in an incubator containing 5% $CO_2$ (v/v) at 37 °C. After 2 weeks, some of the monoclonal wells were selected for amplification into a 24-well plate, followed by amplification into a 6-well plate for culture. The amplified clones were detected and screened by flow cytometry. The results in FIG. 2 show that the selected cell strain 2F8 can stably express human CD25 protein.

**Example 3. Functional Validation of Test Antibodies**

DAC, 7G7B6, Tab06 and 7D4 are all anti-CD25 antibodies. The sequences of DAC are listed in US5530101, which is incorporated herein by reference. 7G7B6 has been proposed as a targeting moiety for the targeting of radionuclides to CD25-expressing lymphomas (Zhang et al, 2009, Cancer Biother Radiopharm 24(3), 303-309). Tab06 is an anti-CD25 antibody that does not inhibit the binding of IL-2 to CD25, the sequences of which are listed in WO2018167104 (sequences 27 and 29 of this patent), which is incorporated herein by reference. 7D4 is a rat IgM anti-mouse CD25 antibody. 7D4 has been widely used for detecting CD25 positive cells in the presence of PC61 or after treatment with PC61 or in the presence of antibodies with similar binding properties (Malek, 1983, Immunology, Vol. 80, pp. 5694-5698; Onizuka S et al., 1999. Canc Res. 59, 3128-3133).

The functional validation was performed on the test antibodies DAC, 7G7B6, Tab06 and 7D4 by FACS. The specific procedures were as follows: SU-DHL-1 cells, CHO-K1 cells, and CHO-K1 cells stably expressing human CD25 (CHO-K1-CD25 cells) were collected in the logarithmic growth phase by centrifugation, washed with PBS, and centrifuged at 200 g for 5 min. The plate was plated with 100 µL of a culture medium containing $2 \times 10^5$ cells, centrifuged at 400 g for 5 min, followed by the addition of the test antibody (100 nM, diluted in a gradient), in which the antibody in the control group was of human IgG isotype. The plate was incubated on ice for 1 h, washed with PBS to remove excess antibody, and centrifuged at 400 g for 5 min. A secondary antibody Alexa Fluor 488 goat anti-human (Fc) antibody (purchased from Life Technology, Cat # A11013) was added for staining for 1 h in an ice bath, and the plate was washed with PBS to remove excess secondary antibody, and centrifuged at 400 g for 5 min. After 200 µL of flow cytometry buffer was added to each well, the plate was assayed using a flow cytometer.

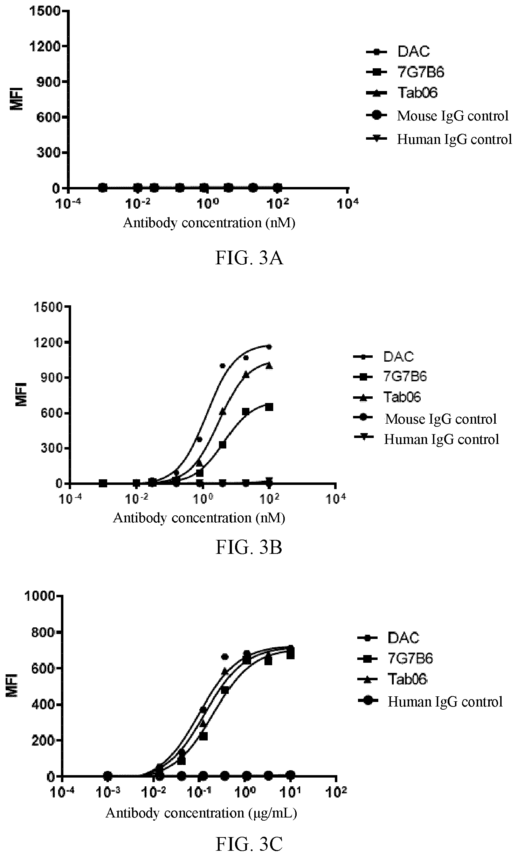

As shown in FIGs. 3A-3C, in the binding experiments with Su-DHL-1 cells and CHO-K1 cells stably expressing human CD25 (CHO-K1-CD25 cells) (i.e., monoclonal 2F8), DAC shows the strongest signal, followed by Tab06, and 7G7B6 shows the weakest signals; the 3 antibodies all can bind to Su-DHL-1 cells and CHO-K1 cells stably expressing human CD25, but not to CHO-K1 cells.

Functional validation was performed on 7G7B6 and Tab06 by ELISA. The specific procedures were as follows: the recombinant human CD25-His protein, the recombinant monkey CD25-His protein and the recombinant mouse CD25-His protein each were diluted to 1 µg/mL with PBS, and the diluted proteins were added into an ELISA microplate at 100 µL/well, and incubated at 4 °C overnight; an ELISA blocking solution (PBS buffer containing 1% BSA, pH 7.4) was added, the plate was blocked at 37 °C for 2 h, test antibodies diluted in a gradient were sequentially added, and the plate was incubated at 37 °C for 1 h and washed for 2-3 times with a washing solution; a horseradish peroxidase-labeled (HRP) secondary antibody was added, and the plate was incubated at 37 °C for 1 h and washed 2-3 times with a washing solution. 100 µL of TMB substrate was added into each well, and after incubation for 15 min at room temperature, 50 µL of stop solution (2M HCl) was added into each well. OD450nm values were read.

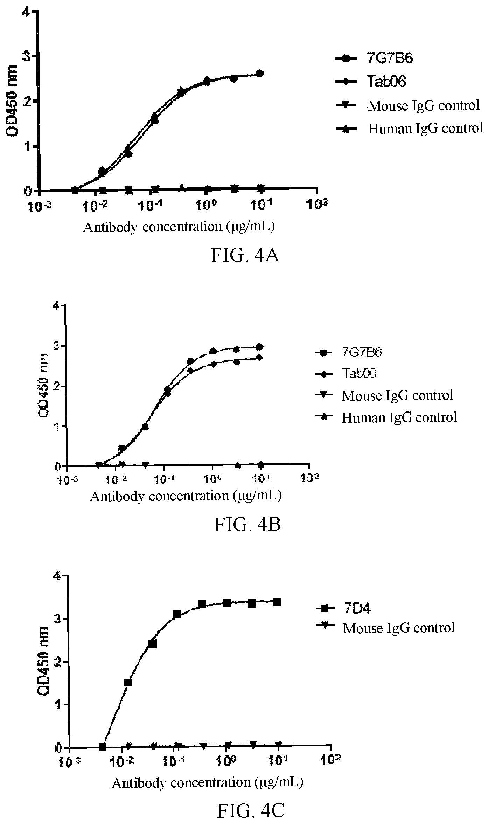

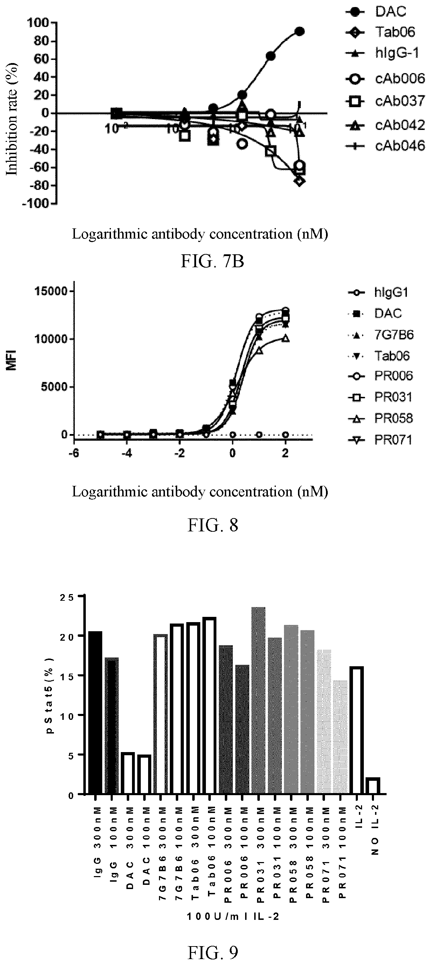

As shown in FIGs. 4A-4C, 7G7B6 and Tab06 can bind to human CD25 protein and monkey CD25 protein, respectively, suggesting that 7G7B6 and Tab06 have human-monkey cross-reactivity; 7D4 can bind to mouse CD25 protein, suggesting that 7D4 has the binding activity against mouse CD25 protein.

**Example 4. Immunization of Mice with Recombinant CD25 Protein to Obtain Murine Anti-CD25 Monoclonal Antibody**

Balb/c and SJL/J mice aged 6-8 weeks were immunized with recombinant human CD25-Fc as an immunogen, and SJL/J mice were immunized with recombinant human CD25-His as an immunogen. The dose for primary immunization was 50 µg per mouse. Two weeks after the primary immunization, booster immunization was performed at an immunization dose of 25 µg per mouse. The subsequent booster immunizations were at an interval of 3 weeks.

Serum samples were collected one week after each booster immunization, and antibody activity in the serum of mice was assayed by ELISA. The plates were coated with 1 µg/mL recombinant human CD25-His, incubated at 4 °C overnight, blocked with a PBST buffer containing 1% BSA for 1 h, and washed 3 times. In a blocking buffer, the serum of mice was diluted in a 10-fold gradient starting at 1:100, and incubated at 37 °C for 1 h. The plate was washed 3 times, and incubated with a secondary antibody against mouse IgG-Fc-HRP for 1 h. The plate was washed 3 times with PBST, and 100 µL of TMB substrate was added to each well. After 15 min, the reaction was terminated with 2M HCl. The absorbance at 450 nm was read using a microplate reader. The serum of mice immunized by the immunogen recombinant human CD25-Fc can bind to the immunogen to different degrees, and still can show antigen-antibody response when the highest dilution factor of the serum is 1:10[5].

In the last immunization, 100 µg of recombinant human CD25-Fc and recombinant human CD25-His was injected intraperitoneally. After 5 days, the mice were sacrificed, from which the spleen was taken and milled to collect spleen cells. The mixed red blood cells in spleen cells were lysed by adding $NH_4OH$ at a final concentration of 1% (w/w) to obtain a spleen cell suspension, which was washed 3 times by centrifugation at 1000 rpm. Mouse spleen cells were mixed with mouse myeloma cells SP2/0 at a 5:1 ratio of number of viable cells, and cell fusion was performed by an efficient electrofusion method. The fused cells were diluted with 20% fetal bovine serum and a DMEM medium containing $1\times$ HAT (w/w), and added into a 96-well culture plate at 200 µL/$1\times10^5$ cells/ well, and the plate was placed in an incubator containing 5% (v/v) $CO_2$ at 37 °C. After 14 days, the fused cells were screened by ELISA, and positive clones with OD450nm > 1.0 were amplified to a 24-well plate, and amplified further at 37 °C, 5% (v/v) $CO_2$ in a DMEM medium containing 10% (w/w) HT fetal bovine serum. After 3 days, the culture medium in the 24-well cell plate was centrifuged to collect the supernatant, and the binding activity against recombinant human CD25 protein and CD25-positive cells was determined by ELISA and FACS. Two rounds of monoclonalization were performed, and excellent monoclonal strains were selected by the above ELISA assay method and flow cytometry. The sequenced amino acid sequences of the antibodies are shown in Table 1, with the complementarity determining region (CDR) sequences underlined (using the Kabat numbering scheme).

Table 1. Sequences of murine anti-CD25 antibodies

| Antibody No. | | Amino acid sequences of heavy chain variable region (VH) and light chain variable region (VL) |
|---|---|---|
| mAb001/ cAb001 | VH | QVTLKESGPGILQPSQTLSLTCSFSGFSLN<u>TFGMGVG</u>WIRQPSGKGLEWLA<u>HIWWDDDKYYNPALKS</u>RLTISKDTSKNQVFLKIANVDTADTVTYYCAR<u>ITTEVGNYDV</u>WGMGTTVTVSS     (SEQ ID NO:1) |
| | VL | EIVLTQSPALMAASPGEKVTITC<u>SVSSGITSNNLH</u>WYQQKSETSPKPWIY<u>GTSNLAS</u>GVPIRFSGSGSGTSYSLTISSMEAEDAATYYC<u>QQWSSYPFT</u>FGSGTKLEIK     (SEQ ID NO:2) |
| mAb002/ cAb002 | VH | QVTLKESGPGILQPSQTLSLTCSFSGFSLS<u>TFGLGVG</u>WIRQPSGKGLEWLA<u>QIWWDDDEYYNPALKS</u>RLTISKDTSRNQVFLKIANVDTGDTATYFCAR<u>MGDTYWYFDV</u>WGTGTTVAVSS     (SEQ ID NO:3) |
| | VL | EIVVTQSPALMAVSPGEKVTITC<u>SLSSGITSSNLH</u>WYQQKSETSPKPWIY<u>GTSNLAS</u>GVPVRFSGSGSKTSYSLTISSMEAEDAATYYC<u>QQWSSYPFT</u>FGSGTKLEIK     (SEQ ID NO:4) |
| mAb004/ cAb004 | VH | EVQLQQSGAELVRPGASVKLSCTASGFNIK<u>DDYIQ</u>WVKQRPEQGLEWIG<u>WIDPENGDTEYASKFQDK</u>ATITDTSSNTAYLQLSSLTSEDTAVYYCAA<u>TTFYASNYGFAY</u>WGQGTLVTVSA     (SEQ ID NO:5) |
| | VL | DVVMTQTPLSLPVSLGDQASISC<u>RSSQSLVHSNGNTYLH</u>WYLQKPGQSPQLLIYK<u>VSNRFS</u>GVPDRFSGSGSGTDFTLKISRLEAEDLGVYFC<u>SQSTHVPYT</u>FGGGTKLEIK     (SEQ ID NO:6) |
| mAb006/ cAb006 | VH | QIQLQQSGAELMKPGASVKLSCKATGYTFT<u>GHWIE</u>WVKQRPGHGLEWVG<u>EILPGSNKINYNEKFKG</u>KATFTAETSSNTVYMQLSSLTTEDSAIYYCAG<u>GGGPHWPFAF</u>WGQGSLVTVSA     (SEQ ID NO:7) |
| | VL | DIVLTQSPASLTVSLGQRATISCR<u>ASESVNVHGAHLMH</u>WYQQKPGQPPKLLIY<u>AASILES</u>GVPARFSGSGSETDFTLNIHPVEEEDAATYFC<u>QQTIEDPRT</u>FGGGTKLEIK     (SEQ ID NO:8) |

(continued)

| Antibody No. | | Amino acid sequences of heavy chain variable region (VH) and light chain variable region (VL) |
| --- | --- | --- |
| mAb028/ cAb028 | VH | EVQLQQSGAELVRPGASVKLSCTASGFNIKNDYVHWVKQRPEQGLEWIGYIDPDNGDTEYASKFLGKATITADTSSNTAYLQLSSLTSEGTAVYYCTTDDTSYGMFACWGLGTLVTVSA (SEQ ID NO:9) |
| | VL | DVLMTQTPLSLPVSLGDQASISCRSSQSIVHSNGNTYLEWYLQKPGQSPKLLIYKVSNRFSGVPDRFSGSGSGTDFTLKISRVEAEDLGVYYCFQGSHVPWTFGGGTKLEIK (SEQ ID NO:10) |
| mAb029/ cAb029 | VH | QVTLKESGPGILQPSQTLSLTCSFSGFSLSTFGMGVGWIRQPSGKGLEWLAHIWWDDDEYYNPALKSRLTISKDTSKNQVFLKIANVDTADTATYYCTRIMLTTGYFDVWGTGTTVTVSS (SEQ ID NO:11) |
| | VL | EIVLTQSPALMAASPGEKVTITCTVSSGITSSNLHWYQQKSETSPKPWIYGTSNLASGVPVRFSGSGSGTSYSLTISSMEAEDAATYYCQQWSSYPFTFGSGTRLEIK (SEQ ID NO:12) |
| mAb037/cAb037 | VH | QVRLQQSGPELVKPGASVQMSCKASGYTFTDYYINWVKQRPGQGLEWIAWIFPGNGNTYFNENFKDKATLSVDKFSSTAFMLLSSLTSEDSAVYFCARATYDHGGYWYFDVWGTGTTVTVSS (SEQ ID NO:13) |
| | VL | QIVLSQSPAILSASPGEKVTMTCRASSSVSHIHWYQQKPGSSPKPWIYATSNLASGVPGRFSGSGSGTSYSLTVSRVEAEDAATYYCQHWNSFTFGGGTELELK (SEQ ID NO:14) |
| mAb042/ cAb042 | VH | QVQLQQSGPELVKSGASVKLSCKASGYTFTTYDINWMKQRPGQGLEWIGWIYPRDGSTKYNAKFKGKATLTEDTSSNTAFMELHSLTSEDSAVYFCARERIYDGSFDYWGQGTTLTVSS (SEQ ID NO:15) |
| | VL | DIVMTQSPSSLSVSAGEKVSMRCKSSQRLFNSRDQKNYLAWYQQKPGQPPKLLIYGASTRESGVPDRFTGSGSGTDFTLTISSVQAEDLAVYYCQNDHIYPLTFGAGTKLELK (SEQ ID NO:16) |
| mAb046/ cAb046 | VH | EVQLVESGGGLVKPGGSLKLSCAASGFTFSSYAMSWVRQTPEKRLEWVATISEGGSYTYYPDNVKGRFTISRDNARNNLYLQMSHLKSEDTAMYLCVRDNPWFAYWGQGTLVTVST (SEQ ID NO:17) |
| | VL | DVLMTQTPLSLPVSLGDRASISCRSSQNIVHSNGNTYLEWFLQKPGQSPKLLIYKVSKRFSGVPDRFSGSGSGTDFTLKISRVEAEDLGVYYCFQGSHVPYTFGGGTKLEIK (SEQ ID NO:18) |

**Example 5. Preparation of Recombinant Chimeric Anti-CD25 Antibody and Humanization Process**

For chimeric antibodies, recombinant chimeric antibodies were obtained by replacing the constant region of the murine monoclonal antibody, and then HEK293 cells were transfected after cloning the nucleotide sequences encoding the variable regions of the murine monoclonal antibody into the pTT5 vector containing protein sequences encoding the human heavy and light chain constant regions (human IgG1, kappa), i.e., the heavy and light chain variable regions of the chimeric antibody obtained were identical to the heavy and light chain variable regions of the murine antibody, respectively. For humanized antibodies, after predicting the structure of the murine monoclonal antibody by homology modeling, the CDRs of the murine antibody were chimerized onto the appropriate human germline framework (Bioinformation. 2014; 10(4): 180-186; Methods MolBiol. 2019;1904:213-230), then back mutations were introduced into sites that could potentially affect antibody-antigen binding, and finally HEK293 cells were transfected after cloning the nucleotide sequences encoding the variable regions of the humanized monoclonal antibody into the pTT5 vector containing protein sequences encoding the human heavy and light chain constant regions (human IgG1, kappa). After 5 days, the cells were removed by centrifugation and the cell culture medium was collected and filtered. After pH was adjusted to 7.0, the collected supernatant of the cell culture medium was loaded on a protein A column (MabSelect SuRe, GE), the bound antibody was eluted with glycine, and the eluate was neutralized with 1M Tris.

The framework selection of variable regions of the antibody is shown in Table 2, the sequences of variable regions of the antibody are shown in Table 3, the CDRs of the heavy chain (HCDR1\HCDR2\HCDR3) and the CDRs of the light chain (LCDR1\LCDR2\LCDR3) are shown in Table 4, and the full length of the amino acid sequences of the antibody is shown in Table 5. PR006 is a humanized antibody of cAb006, PR071 is a humanized antibody of cAb037, PR031 is a humanized antibody of cAb042, and PR058 and PR157 are humanized antibodies of cAb046.

Table 2. Framework selection of variable regions of the antibody

| Antibody No. | VH/VL | Framework 1 (FR1) | Framework 2 (FR2) | Framework 3 (FR3) | Framework 4 (FR4) |
|---|---|---|---|---|---|
| PR006 | VH | IGHV1-46*01 | IGHV1-46*01 | IGHV1-46*01 | IGHJ1*01 |
| | VL | IGKV4-1*01 | IGKV4-1*01 | IGKV4-1*01 | IGKJ4*01 |
| PR071 | VH | IGHV1-18*01 | IGHV1-18*01 | IGHV1-69*02 | hIGHJ6*01_14 |
| | VL | IGKV3-11*01 | IGKV5-2*01 | IGKV6-21*01 | hIGKJ4*01_12 |
| PR031 | VH | IGHV1-18*01 | IGHV4-31*01 | IGHV1-3*01 | hIGHJ6*01 |
| | VL | IGKV4-1*01 | IGKV4-1*01 | IGKV4-1*01 | hIGKJ2*01 |
| PR058/ PR157 | VH | IGHV3-23*04 | IGHV3-23*04 | IGHV3-23*04 | IGHJ1*01 |
| | VL | IGKV2-28*01 | IGKV2-28*01 | IGKV2-28*01 | IGKJ4*01 |

Table 3. Sequences of variable regions of some of humanized anti-CD25 antibodies not blocking IL-2 function

| Antibody No. | | Amino acid sequences of heavy chain variable region (VH) and light chain variable region (VL) |
|---|---|---|
| PR006 | VH | EVQLVQSGAEVKKPGASVKVSCKASGYTFTGHWIEWVRQAPGQGLE WMGEILPGSNKINYNEKFKGRVTFTADTSTSTVYMELSSLRSEDTAVY YCAGGGGPHWPFAFWGQGTLVTVSS     (SEQ ID NO:19) |
| | VL | DIVMTQSPDSLAVSLGERATINCRASESVNVHGAHLMHWYQQKPGQP PKLLIYAASILESGVPDRFSGSGSGTDFTLTISSLQAEDVAVYYCQQTIE DPRTFGGGTKVEIK     (SEQ ID NO:20) |
| PR071 | VH | EVQLVQSGSELKKPGASVKVSCKASGYTFTDYYINWVRQAPGQGLE WMAWIFPGNGNTYFNENFKDRVTLTADKFSSTAYMELSSLRSEDTAV YYCARATYDHGGYWYFDVWGQGTTVTVSS     (SEQ ID NO:21) |
| | VL | EIVLSQSPATLSLSPGERATLSCRASSSVSHIHWYQQKPGSSPKPWIYAT SNLASGVPGRFSGSGSGTSYSLTVSRVEAEDAATYYCQHWNSFTFGG GTKVEIK     (SEQ ID NO:22) |
| PR031 | VH | EVQLVQSGAEVKKPGASVKVSCKASGYTFTTYDINWMKQHPGKGLE WIGWIYPRDGSTKYNAKFKGKATLTEDTSANTAYMELSSLRSEDTAVY YCARERIYDGSFDYWGQGTTVTVSS     (SEQ ID NO:23) |
| | VL | DIVMTQSPDSLAVSLGERATINCKSSQRLFNSRDQKNYLAWYQQKPG QPPKLLIYGASTRESGVPDRFSGSGSGTDFTLTISSLQAEDVAVYYCQN DHIYPLTFGQGTKLEIK     (SEQ ID NO:24) |

(continued)

| Antibody No. | Amino acid sequences of heavy chain variable region (VH) and light chain variable region (VL) | |
|---|---|---|
| PR058 | VH | EVQLVESGGGLVQPGGSLRLSCAASGFTFSSYAMSWVRQAPGKRLEWVATISEGGSYTYYPDNVKGRFTISRDNSKNTLYLQMNSLRAEDTAVYLCVRDNPWFAYWGQGTLVTVSS    (SEQ ID NO:25) |
| | VL | DVLMTQSPLSLPVTPGEPASISCRSSQNIVHSNGNTYLEWFLQKPGQSPKLLIYKVSKRFSGVPDRFSGSGSGTDFTLKISRVEAEDVGVYYCFQGSHVPYTFGGGTKVEIK    (SEQ ID NO:26) |
| PR157 | VH | EVQLVESGGGLVQPGGSLRLSCAASGFTFSSYAMSWVRQAPGQRLEWVATISEGGSYTYYPDNVKGRFTISRDNSKNTLYLQMNSLRAEDTAVYLCVRDNPWFAYWGQGTLVTVSS    (SEQ ID NO:59) |
| | VL | DVLMTQSPLSLPVTPGEPASISCRSSQNIVHSNGNTYLEWFLQKPGQSPKLLIYKVSKRFSGVPDRFSGSGSGTDFTLKISRVEAEDVGVYYCFQGSHVPYTFGGGTKVEIK    (SEQ ID NO:60) |

Table 4. Sequences of variable regions of some of humanized anti-CD25 antibodies not blocking IL-2 function (Kabat numbering scheme)

| Antibody No. | Heavy chain variable region CDR | | Light chain variable region CDR | |
|---|---|---|---|---|
| PR006 | HCDR1 | GHWIE (SEQ ID NO:27) | LCDR1 | RASESVNVHGAHLMH (SEQ ID NO:30) |
| | HCDR2 | EILPGSNKINYNEKFKG (SEQ ID NO:28) | LCDR2 | AASILES (SEQ ID NO:31) |
| | HCDR3 | GGGPHWPFAF (SEQ ID NO:29) | LCDR3 | QQTIEDPRT (SEQ ID NO:32) |
| PR071 | HCDR1 | DYYIN (SEQ ID NO:33) | LCDR1 | RASSSVSHIH (SEQ ID NO:36) |
| | HCDR2 | WIFPGNGNTYFNENFKD (SEQ ID NO:34) | LCDR2 | ATSNLAS (SEQ ID NO:37) |
| | HCDR3 | ATYDHGGYWYFDV (SEQ ID NO:35) | LCDR3 | QHWNSFT (SEQ ID NO:38) |
| PR031 | HCDR1 | TYDIN (SEQ ID NO:39) | LCDR1 | KSSQRLFNSRDQKNYLA (SEQ ID NO:42) |
| | HCDR2 | WIYPRDGSTKYNAKFKG (SEQ ID NO:40) | LCDR2 | GASTRES (SEQ ID NO:43) |
| | HCDR3 | ERIYDGSFDY (SEQ ID NO:41) | LCDR3 | QNDHIYPLT (SEQ ID NO:44) |
| PR058 | HCDR1 | SYAMS (SEQ ID NO:45) | LCDR1 | RSSQNIVHSNGNTYLE (SEQ ID NO:48) |
| | HCDR2 | TISEGGSYTYYPDNVKG (SEQ ID NO:46) | LCDR2 | KVSKRFS (SEQ ID NO:49) |
| | HCDR3 | DNPWFAY (SEQ ID NO:47) | LCDR3 | FQGSHVPYT (SEQ ID NO:50) |
| PR157 | HCDR1 | SYAMS (SEQ ID NO:45) | LCDR1 | RSSQNIVHSNGNTYLE (SEQ ID NO:48) |
| | HCDR2 | TISEGGSYTYYPDNVKG (SEQ ID NO:46) | LCDR2 | KVSKRFS (SEQ ID NO:49) |
| | HCDR3 | DNPWFAY (SEQ ID NO:47) | LCDR3 | FQGSHVPYT (SEQ ID NO:50) |

Table 5. Full length amino acid sequences of heavy and light chains of humanized antibodies

| Antibody No. | | Full length amino acid sequences of heavy and light chains |
|---|---|---|
| PR006 | Heavy chain | EVQLVQSGAEVKKPGASVKVSCKASGYTFT<u>GHWIE</u>WVRQAPGQGL EWMG<u>EILPGSNKINYNEKFKG</u>RVTFTADTSTSTVYMELSSLRSEDTA VYYCAGG<u>GGGPHWPFAF</u>WGQGTLVTVSSASTKGPSVFPLAPSSKSTS GGTAALGCLVKDYFPEPVTVSWNSGALTSGVHTFPAVLQSSGLYSLS SVVTVPSSSLGTQTYICNVNHKPSNTKVDKKVEPKSCDKTHTCPPCP APELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSREEMTKNQVSLTCLVK GFYPSDIAVEWESNGQPENNYKTTPPVLDSDGSFFLYSKLTVDKSRW QQGNVFSCSVMHEALHNHYTQKSLSLSPGK    (SEQ ID NO:51) |
| | Light chain | DIVMTQSPDSLAVSLGERATINC<u>RASESVNVHGAHLMH</u>WYQQKPGQ PPKLLIY<u>AASILES</u>GVPDRFSGSGSGTDFTLTISSLQAEDVAVYYC<u>QQTI EDPRT</u>FGGGTKVEIKRTVAAPSVFIFPPSDEQLKSGTASVVCLLNNFY PREAKVQWKVDNALQSGNSQESVTEQDSKDSTYSLSSTLTLSKADY EKHKVYACEVTHQGLSSPVTKSFNRGEC    (SEQ ID NO:52) |
| PR071 | Heavy chain | EVQLVQSGSELKKPGASVKVSCKASGYTFT<u>DYYIN</u>WVRQAPGQGLE WMA<u>WIFPGNGNTYFNENFKD</u>RVTLTADKFSSTAYMELSSLRSEDTAV YYCAR<u>ATYDHGGYWYFDV</u>WGQGTTVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSREEMTKNQVSLTCL VKGFYPSDIAVEWESNGQPENNYKTTPPVLDSDGSFFLYSKLTVDKS RWQQGNVFSCSVMHEALHNHYTQKSLSLSPGK    (SEQ ID NO:53) |
| | Light chain | EIVLSQSPATLSLSPGERATLSC<u>RASSSVSHIH</u>WYQQKPGSSPKPWIY<u>A TSNLAS</u>GVPGRFSGSGSGTSYSLTVSRVEAEDAATYYC<u>QHWNSFT</u>FG GGTKVEIKRTVAAPSVFIFPPSDEQLKSGTASVVCLLNNFYPREAKVQ WKVDNALQSGNSQESVTEQDSKDSTYSLSSTLTLSKADYEKHKVYA CEVTHQGLSSPVTKSFNRGEC    (SEQ ID NO:54) |

(continued)

| Antibody No. | | Full length amino acid sequences of heavy and light chains |
|---|---|---|
| PR031 | Heavy chain | EVQLVQSGAEVKKPGASVKVSCKASGYTFT<u>TYDIN</u>WMKQHPGKGL EWIG<u>WIYPRDGSTKYNAKFKG</u>KATLTEDTSANTAYMELSSLRSEDTA VYYCAR<u>ERIYDGSFDY</u>WGQGTTVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPA PELLGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPSREEMTKNQVSLTCLVKG FYPSDIAVEWESNGQPENNYKTTPPVLDSDGSFFLYSKLTVDKSRWQ QGNVFSCSVMHEALHNHYTQKSLSLSPGK    (SEQ ID NO:55) |
| | Light chain | DIVMTQSPDSLAVSLGERATINC<u>KSSQRLFNSRDQKNYLA</u>WYQQKPG QPPKLLIY<u>GASTRES</u>GVPDRFSGSGSGTDFTLTISSLQAEDVAVYYC<u>Q NDHIYPLT</u>FGQGTKLEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC    (SEQ ID NO:56) |
| PR058 | Heavy chain | EVQLVESGGGLVQPGGSLRLSCAASGFTFS<u>SYAMS</u>WVRQAPGKRLE WVA<u>TISEGGSYTYYPDNVKG</u>RFTISRDNSKNTLYLQMNSLRAEDTAV YLCVR<u>DNPWFAY</u>WGQGTLVTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKAL PAPIEKTISKAKGQPREPQVYTLPPSREEMTKNQVSLTCLVKGFYPSDI AVEWESNGQPENNYKTTPPVLDSDGSFFLYSKLTVDKSRWQQGNVF SCSVMHEALHNHYTQKSLSLSPGK    (SEQ ID NO:57) |
| | Light chain | DVLMTQSPLSLPVTPGEPASISC<u>RSSQNIVHSNGNTYLE</u>WFLQKPGQS PKLLIY<u>KVSKRFS</u>GVPDRFSGSGSGTDFTLKISRVEAEDVGVYYC<u>FQ GSHVPYT</u>FGGGTKVEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC    (SEQ ID NO:58) |

(continued)

| Antibody No. | | Full length amino acid sequences of heavy and light chains |
|---|---|---|
| PR0157 | Heavy chain | EVQLVESGGGLVQPGGSLRLSCAASGFTFSSYAMSWVRQAPGQRLE WVATISEGGSYTYYPDNVKGRFTISRDNSKNTLYLQMNSLRAEDTAV YLCVRDNPWFAYWGQGTLVTVSSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKAL PAPIEKTISKAKGQPREPQVYTLPPSREEMTKNQVSLTCLVKGFYPSDI AVEWESNGQPENNYKTTPPVLDSDGSFFLYSKLTVDKSRWQQGNVF SCSVMHEALHNHYTQKSLSLSPGK (SEQ ID NO:61) |
| | Light chain | DVLMTQSPLSLPVTPGEPASISCRSSQNIVHSNGNTYLEWFLQKPGQS PKLLIYKVSKRFSGVPDRFSGSGSGTDFTLKISRVEAEDVGVYYCFQ GSHVPYTFGGGTKVEIKRTVAAPSVFIFPPSDEQLKSGTASVVCLLNN FYPREAKVQWKVDNALQSGNSQESVTEQDSKDSTYSLSSTLTLSKA DYEKHKVYACEVTHQGLSSPVTKSFNRGEC (SEQ ID NO:62) |

**Example 6. Assay on Affinity of Chimeric Anti-CD25 Antibodies for Human and Cynomolgus Monkey (Cyno) CD25 Proteins**

The binding activity of the chimeric anti-CD25 antibody and the CD25 recombinant protein was assayed by ELISA. The 96-well plate was coated with human CD25-His/monkey CD25-His at a concentration of 1 g/mL at 50 μL/well. The unbound antigen was washed away with a washing solution (1× PBST). After washing, the plate was blocked with 1% BSA blocking solution prepared in 1× PBST at 37 °C for 1 h. After washing 3 times with the washing solution, the test chimeric antibodies at different dilution concentrations was added and incubated in an incubator at 37 °C for 1 h. After washing 3 times with the washing solution, 100 μL of goat anti-human IgG secondary antibody diluted at 1:5000 was added and incubated in an incubator at 37 °C for 0.5 h. The plate were washed, the TMB color developing solutions A and B were mixed in a 1:1 ratio for color development. After 15 min, the color development reaction was terminated with 1M hydrochloric acid, and the fluorescence value at 450 nm was measured. The results show that the test chimeric anti-CD25 antibodies all can bind to human and monkey CD25 proteins. The median effective concentrations (EC$_{50}$) by the ELISA assay are shown in Tables 6 and 7.

Table 6. EC$_{50}$ for binding of chimeric anti-CD25 antibodies to monkey CD25 (ELISA)

| Antibody No. | EC$_{50}$(nM) | Antibody No. | EC$_{50}$(nM) | Antibody No. | EC$_{50}$(nM) |
|---|---|---|---|---|---|
| cAb001 | 0.3991 | cAb029 | 0.1145 | cAb028 | 0.2295 |
| cAb002 | 0.5244 | cAb037 | 0.1467 | DAC | 0.3973 |
| cAb004 | 0.3106 | cAb042 | 0.3486 | Tab06 | 0.6144 |
| cAb006 | 0.2338 | cAb046 | 5.569 | | |

Table 7. EC$_{50}$ for binding of chimeric anti-CD25 antibodies to human CD25 (ELISA)

| Antibody No. | EC$_{50}$(nM) | Antibody No. | EC$_{50}$(nM) | Antibody No. | EC$_{50}$(nM) |
|---|---|---|---|---|---|
| cAb001 | 0.2284 | cAb028 | 0.3444 | cAb046 | 0.3547 |
| cAb002 | 0.2738 | cAb029 | 0.1661 | DAC | 0.2319 |
| cAb004 | 0.2552 | cAb037 | 0.1653 | Tab06 | 0.4032 |
| cAb006 | 0.2004 | cAb042 | 0.224 | | |

## Example 7. Binding of Chimeric Anti-CD25 Antibodies to SU-DHL-1 Cells

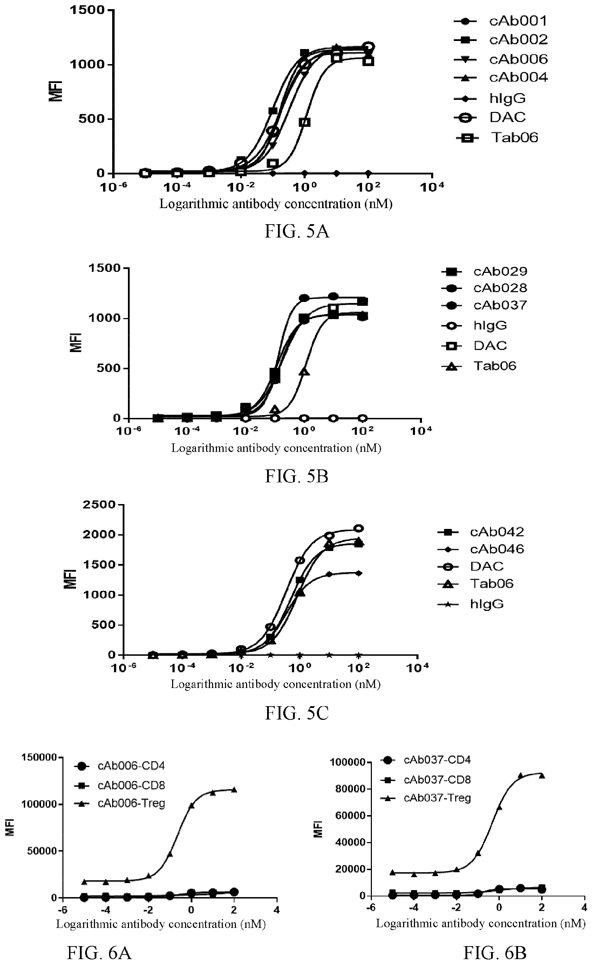

Since the lymphoma cell line SU-DHL-1 highly expresses CD25, the binding of chimeric anti-CD25 antibodies to SU-DHL-1 cells was determined by flow cytometry. SU-DHL-1 cells in logarithmic growth phase were selected, collected, washed with PBS, and centrifuged. Each well was plated with 100 μL of $2\times10^5$ cells and centrifuged at 400 g for 5 min. The test antibodies at different concentrations were added, incubated on ice for 1 h, washed with PBS, and centrifuged at 400 g for 5 min. The goat anti-human secondary antibody Alexa Fluor 488 with a fluorophore was added for staining for 1 h in an ice bath, and the plate was washed with PBS, and assayed using a flow cytometer. As shown in FIGs. 5A-5C, the test chimeric anti-CD25 antibodies of the present disclosure all can bind to SU-DHL-1 cells. Table 8 shows the $EC_{50}$ values for binding.

Table 8. $EC_{50}$ for binding of chimeric anti-CD25 antibodies to SU-DHL-1 cells

| Antibody No. | $EC_{50}$(nM) | Antibody No. | $EC_{50}$(nM) | Antibody No. | $EC_{50}$(nM) |
|---|---|---|---|---|---|
| cAb001 | 0.1799 | cAb028 | 0.1319 | cAb046 | 0.3455 |
| cAb002 | 0.1001 | cAb029 | 0.1193 | DAC | 0.3407 |
| cAb004 | 0.1681 | cAb037 | 0.1502 | Tab06 | 0.8323 |
| cAb006 | 0.3286 | cAb042 | 0.501 | | |

## Example 8. Binding of Chimeric Anti-CD25 Antibodies to Treg Cells/Activated CD4+ T Cells/CD8+ T Cells

Since CD25 is expressed in both activated Tregs and activated effector T cells, in order to test whether the anti-CD25 antibodies of the present disclosure bind differentially to CD25 expressed on Tregs and CD4+ and CD8+ effector T cells, the binding ability of the antibodies to Tregs and activated effector T cells was compared by the FACS method below.

Natural CD4+ cells were isolated from human peripheral blood mononuclear cells (PBMCs) by the magnetic bead method, and a Treg-inducing medium (AIM V + 10% FBS + human IL-2 (400 U/mL) + Rapamycin (0.1 g/mL) or TGFβ (20 ng/mL) + atRA (200 nM)) was added together with magnetic beads coated with the anti-CD3/CD28 antibody. The cell density was maintained at $2\times10^6$/mL during the culture at 37 °C, 5% $CO_2$. After culturing for 7 days, activated CD4+CD25+ Treg cells were obtained by isolating cells with Dynabeads™ Regulatory CD4+/CD25+ T Cell Kit (Thermo Fisher, 11363D), and Treg cells with FOXP3 positive rate > 90% were obtained.

Fresh PBMCs were enriched with CD4+ cell negative screening and enriching kit and CD8+ T cell screening kit, and isolated to obtain highly pure CD4+ and CD8+ effector T cells, with cell purity greater than 90%. In addition, magnetic beads coated with an anti-CD3/CD28 antibody were added to activate and expand the cells. After 3 days, effector CD4+ T cells and effector CD8+ T cells with high expression of CD25 were obtained. Treg cells induced *in vitro* and CD4+ and CD8+ effector T cells activated *in vitro* each were added to the test antibodies at different dilution concentrations, incubated on ice for 1 h, and then washed with PBS. Then, a goat anti-human antibody Alexa Fluor 488 was added for staining for 1 h in an ice bath, and the plate was washed with PBS, followed by the addition of 200 μL of flow cytometry buffer to each well for simultaneous assay using a flow cytometer under the same condition.

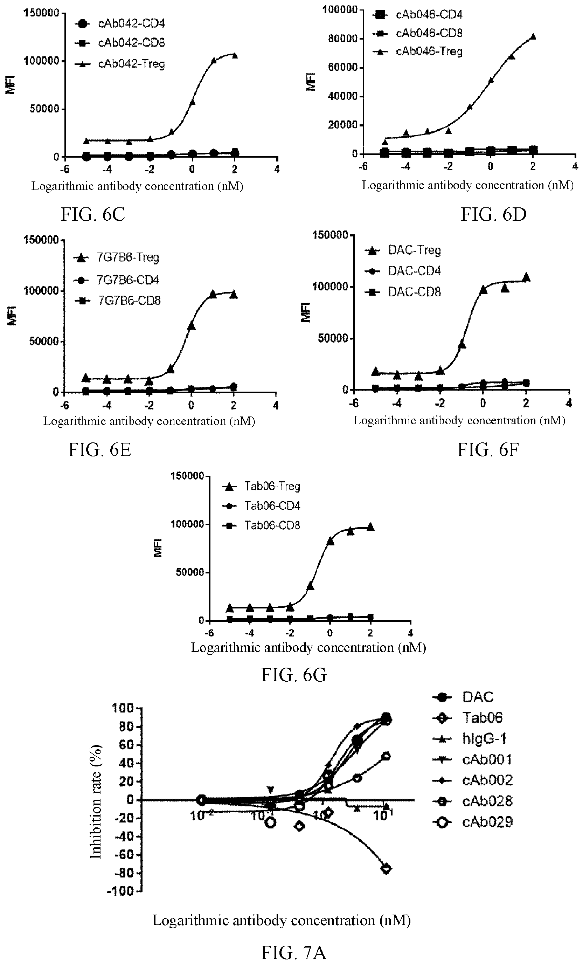

The results are shown in FIGs. 6A-6G, and it can be seen that the test chimeric antibodies all can bind to CD25 antigens on Treg cells and activated CD4+ and CD8+ effector T cells. However, the binding ability to Tregs is much greater than that to CD4+ T cells, and the binding ability to CD4+ T cells is slightly greater than that to CD8+ T cells, in terms of the extent of binding.

## Example 9. Effect of Chimeric Anti-CD25 Antibodies on the Binding Ability of IL-2 to the Receptor

SU-DHL-1 cells in logarithmic growth phase were collected, incubated with 200 nM IL-2-biotin and test chimeric anti-CD25 antibodies at different concentrations at 4 °C for 1 h, and washed once with PBS. Alexa Fluor 488 Streptavidin (1: 1000) was added, and the cells were incubated at 4 °C for 1 h and washed with PBS to remove excess secondary antibody. The fluorescent cells were detected with FACS. If the antibody affects the binding of IL-2 to CD25, the fluorescence intensity becomes weaker.

The inhibition rate was calculated according to the following formula:

Inhibition rate % = 100 × (maximum binding fluorescence intensity without antibody addition - fluorescence intensity after antibody binding) / binding intensity without antibody addition.

The results are shown in FIGs. 7A-7B, and it can be seen that DAC, cAb001, cAb002, cAb028 and cAb029 inhibit the binding of IL-2 to SU-DHL-1 to different degrees, suggesting that the above chimeric antibodies inhibit the IL-2 signaling pathway, while cAb006, cAb037, cAb042 and cAb046 do not inhibit the binding of IL-2 to SU-DHL-1, as with Tab006, suggesting that the above chimeric antibodies are non-antagonistic antibodies.

**Example 10. Binding of Humanized Anti-CD25 Antibodies to SUDH-L1 Cells**

The binding of chimeric anti-CD25 antibodies to SU-DHL-1 cells was assayed by flow cytometry as described in Example 7. FIG. 8 shows that the humanized anti-CD25 antibodies of the present disclosure (RP006, PR031, PR058 and PR071) all have good SU-DHL-1-binding activity.

**Example 11. Effect of Humanized Anti-CD25 Antibodies on the Human Peripheral Blood T lymphocyte pStat5 Signaling Pathway**

CD25, as the $\alpha$ chain of the IL-2 cytokine receptor, forms a high-affinity receptor with $\beta$ and $\gamma$ chains, and activates the downstream JAK-STAT, PI3K-AKT and MAPK signaling pathways after binding to IL-2. Therefore, the effect of chimeric antibodies on the up-regulation of phosphorylated STAT-5 by IL-2 in PBMC was further tested.

The cryopreserved PBMCs were thawed and co-incubated with the humanized anti-CD25 antibody (37 °C, 5% $CO_2$). After 30 min, 100U/mL IL-2 was added to induce the cells for 10 min. Activated PBMCs were fixed, 200 $\mu$L of staining solution (PBS containing 2% FBS) was added into each well, followed by the addition of 5 $\mu$L of V450 mouse anti-human CD3 labeled T cells. After unbound antibody was washed away, 300 $\mu$L of precooled permeabilization reagent Perm Buffer III was added, and the mixture was mixed uniformly by vortexing, and left to stand in an ice bath for 30 min. Then, Alexa Fluor® 647 mouse anti-Stat5 was added for staining for 60 min. Excess antibody was removed, and assay was performed by flow cytometry.

As shown in FIG. 9, DAC can significantly inhibit IL-2-induced phosphorylation of Stat5 in CD3 cells, whereas Tab06 cannot inhibit IL-2-induced phosphorylation of Stat5. PR006 can weakly inhibit the phosphorylation of Stat5 at 300 nM and 100 nM, and PR031, PR058 and PR071 do not inhibit IL-2-mediated phosphorylation of Stat5.

**Example 12. ADCC Effect on SU-DHL-1 Cells Mediated by Humanized Anti-CD25 Antibodies**

Antibody-dependent cell-mediated cytotoxicity (ADCC) is an important function of antibody therapy, and it is expected that the activation of tumor immunity is achieved by killing Treg cells highly expressing CD25 through the activity of ADCC. The activity of ADCC mediated by the humanized anti-CD25 antibodies of the present disclosure was assayed as follows.

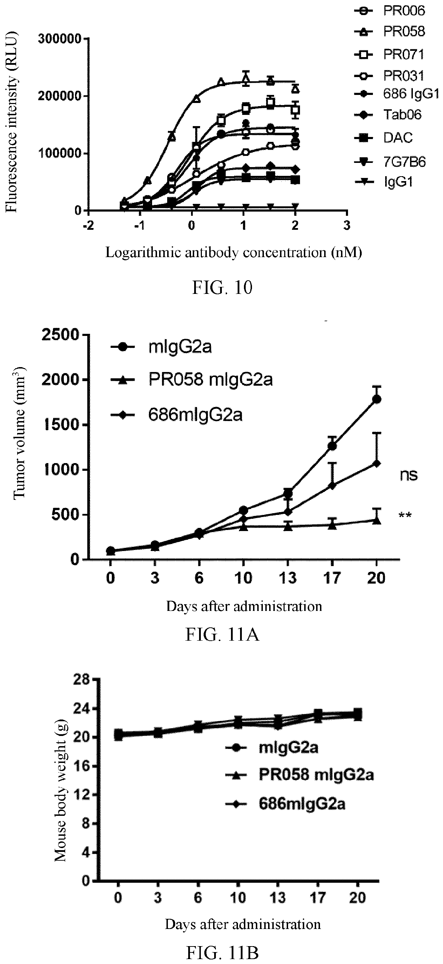

ADCC reporter gene cells (Promega G7010 ADCC Bioassay Effector Cells) were mixed with SU-DHL-1 cells in a 1:1 ratio, and after antibodies at different concentrations were added for incubation for 6 h, the fluorescence activity of the cells was assayed. It can be seen from the FIG. 10 and Table 9 that PR006, PR031, PR058 and PR071 have good ADCC activity, wherein PR058 and PR071 have significantly stronger activity than the antibody 686.

The sequences of the antibody 686 can be found in sequence 5 and sequence 9 of US20190284287A.

Table 9. ADCC activity of humanized antibodies

| Antibody No. | PR006 | PR058 | PR071 | PR031 | DAC | Tab06 | 7G7B6 | 686 |
|---|---|---|---|---|---|---|---|---|
| $EC_{50}$ | 0.5153 | 0.3421 | 0.9134 | 1.034 | 0.8194 | 1.347 | 1.09 | 0.8366 |
| Multiple of maximum fluorescence value (vs. IgG) | 239.2 | 385.7 | 322 | 192.2 | 103 | 132.9 | 13.288 | 249.3 |

**Example 13. Anti-Tumor Activity Against an MC38 Xenograft Tumor Model of hCD25 Mice Mediated by Humanized Anti-CD25 Antibodies**

The anti-tumor activity of the humanized anti-CD25 antibodies was evaluated in an MC38 colon cancer mouse model of B-hIL2RA humanized mice. The variable regions of the above PR058 and 686 were connected to mouse IgG2a to construct PR058 mIgG2a and 686 mIgG2a antibodies for animal experiments.

MC38 colon cancer cells suspended in PBS were inoculated subcutaneously on the right side of B-hIL2RA humanized mice at a concentration of $5\times10^5/0.1$ mL, in a volume of 0.1 mL/mouse. When the mean tumor volume reached approximately 113 mm$^3$, 48 mice with appropriate individual tumor volumes were selected and randomly divided into 6 groups with 8 mice in each group, including: a G1 isotype mIgG2a (3 mg/kg) control group, a G2 PR058 mIgG2a (3 mg/kg) group, and a G3 686 mIgG2a (3 mg/kg) group. All groups were administered by intraperitoneal injection once a week for 3 times, and the experiment ended 7 days after the last administration. Tumor volume and body weight of mice were measured and recorded twice a week. At the end of the experiment, the animals were euthanized, tumors were taken, weighed, and photographed, and the relative tumor inhibition (TGI%) was calculated.

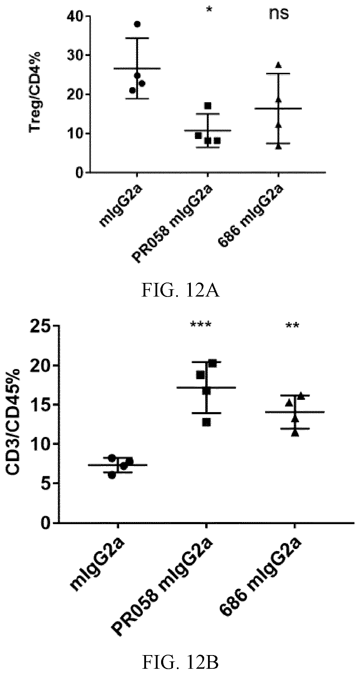

The study results are shown in FIG. 11A, and it can be seen that PR058 has the best inhibition effect on the growth of MC38 subcutaneous xenograft tumor at the dose level of 3 mg/kg, and it is superior to 686. FIG. 11B shows that the experimental animals have good activity and feeding status during the treatment, and the body weight of the experimental animals increases to some degrees, suggesting that the test drugs do not produce significant toxic effects on the experimental animals, and have better safety. The analysis results of the intratumoral lymphocyte in FIGs. 12A and 12B show that PR058 can mediate Treg killing and up-regulation of the number of CD3 more strongly. * indicates $p < 0.05$, with statistical difference compared to the mIgG2a group; ** indicates $p < 0.01$, with significant statistical difference compared to the mIgG2a group; *** indicates $p < 0.001$, with extremely significant statistical difference compared to the mIgG2a group; ns indicates no statistical difference compared to the mIgG2a group. Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

**Claims**

1. An anti-CD25 antibody or an antigen-binding fragment thereof, comprising a heavy chain variable region (VH) and a light chain variable region (VL), wherein:

   1) the VH comprises an HCDR1, an HCDR2 and an HCDR3 in SEQ ID NO: 17, and the VL comprises an LCDR1, an LCDR2 and an LCDR3 in SEQ ID NO: 18;
   2) the VH comprises an HCDR1, an HCDR2 and an HCDR3 in SEQ ID NO: 1, and the VL comprises an LCDR1, an LCDR2 and an LCDR3 in SEQ ID NO: 2;
   3) the VH comprises an HCDR1, an HCDR2 and an HCDR3 in SEQ ID NO: 3, and the VL comprises an LCDR1, an LCDR2, and an LCDR3 in SEQ ID NO: 4;
   4) the VH comprises an HCDR1, an HCDR2 and an HCDR3 in SEQ ID NO: 5, and the VL comprises an LCDR1, an LCDR2 and an LCDR3 in SEQ ID NO: 6;
   5) the VH comprises an HCDR1, an HCDR2 and an HCDR3 in SEQ ID NO: 7, and the VL comprises an LCDR1, an LCDR2 and an LCDR3 in SEQ ID NO: 8;
   6) the VH comprises an HCDR1, an HCDR2 and an HCDR3 in SEQ ID NO: 9, and the VL comprises an LCDR1, an LCDR2, and an LCDR3 in SEQ ID NO: 10;
   7) the VH comprises an HCDR1, an HCDR2 and an HCDR3 in SEQ ID NO: 11, and the VL comprises an LCDR1, an LCDR2 and an LCDR3 in SEQ ID NO: 12;
   8) the VH comprises an HCDR1, an HCDR2 and an HCDR3 in SEQ ID NO: 13, and the VL comprises an LCDR1, an LCDR2 and an LCDR3 in SEQ ID NO: 14; or
   9) the VH comprises an HCDR1, an HCDR2 and an HCDR3 in SEQ ID NO: 15, and the VL comprises an LCDR1, an LCDR2 and an LCDR3 in SEQ ID NO: 16;

   wherein the CDRs are defined according to the Kabat, IMGT, Chothia, AbM or Contact numbering scheme; preferably, the CDRs are defined according to the Kabat numbering scheme.

2. An anti-CD25 antibody or an antigen-binding fragment thereof, comprising a VH and a VL, wherein:

   1) the VH comprises an HCDR1, an HCDR2 and an HCDR3 set forth in SEQ ID NOs: 45, 46 and 47, respectively, and the VL comprises an LCDR1, an LCDR2 and an LCDR3 set forth in SEQ ID NOs: 48, 49 and 50, respectively;
   2) the VH comprises an HCDR1, an HCDR2 and an HCDR3 set forth in SEQ ID NOs: 27, 28 and 29, respectively, and the VL comprises an LCDR1, an LCDR2 and an LCDR3 set forth in SEQ ID NOs: 30, 31 and 32, respectively;

3) the VH comprises an HCDR1, an HCDR2 and an HCDR3 set forth in SEQ ID NOs: 33, 34 and 35, respectively, and the VL comprises an LCDR1, an LCDR2 and an LCDR3 set forth in SEQ ID NOs: 36, 37 and 38, respectively; or

4) the VH comprises an HCDR1, an HCDR2 and an HCDR3 set forth in SEQ ID NOs: 39, 40 and 41, respectively, and the VL comprises an LCDR1, an LCDR2 and an LCDR3 set forth in SEQ ID NOs: 42, 43 and 44, respectively.

3. The anti-CD25 antibody or the antigen-binding fragment thereof according to any one of claims 1 to 2, wherein the anti-CD25 antibody is a murine antibody, a chimeric antibody, a human antibody, or a humanized antibody; preferably, the anti-CD25 antibody is a humanized antibody.

4. The anti-CD25 antibody or the antigen-binding fragment thereof according to any one of claims 1 to 2, wherein:

1) the VH comprises an FR1 to an FR3 selected from IGHV1-46*01, and an FR4 selected from IGHJ1*01, and the VL comprises an FR1 to an FR3 selected from IGKV4-1*01, and an FR4 selected from IGKJ4*01;

2) the VH comprises an FR1 to an FR2 selected from IGHV1-18*01, an FR3 selected from IGHV1-69*02, and an FR4 selected from hIGHJ6*01_14, and the VL comprises an FR1 selected from IGKV3-11*01, an FR2 selected from IGKV5-2*01, an FR3 selected from IGKV6-21*01, and an FR4 selected from hIGKJ4*01_12;

3) the VH comprises an FR1 selected from IGHV1-18*01, an FR2 selected from IGHV4-31*01, an FR3 selected from IGHV1-3*01, and an FR4 selected from hIGHJ6*01, and the VL comprises an FR1 to an FR3 selected from IGKV4-1*01, and an FR4 selected from hIGKJ2*01; or

4) the VH comprises an FR1 to an FR3 selected from IGHV3-23*04, and an FR4 selected from IGHJ1*01, and the VL comprises an FR1 to an FR3 selected from IGKV2-28*01, and an FR4 selected from IGKJ4*01.

5. The anti-CD25 antibody or the antigen-binding fragment thereof according to any one of claims 1 to 4, wherein:

the VH is set forth in SEQ ID NO: 25 or has at least 90% or at least 95% sequence identity thereto, and the VL is set forth in SEQ ID NO: 26 or has at least 90% or at least 95% sequence identity thereto;

the VH is set forth in SEQ ID NO: 59 or has at least 90% or at least 95% sequence identity thereto, and the VL is set forth in SEQ ID NO: 60 or has at least 90% or at least 95% sequence identity thereto;

the VH is set forth in SEQ ID NO: 1 or has at least 90% or at least 95% sequence identity thereto, and the VL is set forth in SEQ ID NO: 2 or has at least 90% or at least 95% sequence identity thereto;

the VH is set forth in SEQ ID NO: 3 or has at least 90% or at least 95% sequence identity thereto, and the VL is set forth in SEQ ID NO: 4 or has at least 90% or at least 95% sequence identity thereto;

the VH is set forth in SEQ ID NO: 5 or has at least 90% or at least 95% sequence identity thereto, and the VL is set forth in SEQ ID NO: 6 or has at least 90% or at least 95% sequence identity thereto;

the VH is set forth in SEQ ID NO: 7 or has at least 90% or at least 95% sequence identity thereto, and the VL is set forth in SEQ ID NO: 8 or has at least 90% or at least 95% sequence identity thereto;

the VH is set forth in SEQ ID NO: 9 or has at least 90% or at least 95% sequence identity thereto, and the VL is set forth in SEQ ID NO: 10 or has at least 90% or at least 95% sequence identity thereto;

the VH is set forth in SEQ ID NO: 11 or has at least 90% or at least 95% sequence identity thereto, and the VL is set forth in SEQ ID NO: 12 or has at least 90% or at least 95% sequence identity thereto;

the VH is set forth in SEQ ID NO: 13 or has at least 90% or at least 95% sequence identity thereto, and the VL is set forth in SEQ ID NO: 14 or has at least 90% or at least 95% sequence identity thereto;

the VH is set forth in SEQ ID NO: 15 or has at least 90% or at least 95% sequence identity thereto, and the VL is set forth in SEQ ID NO: 16 or has at least 90% or at least 95% sequence identity thereto;

the VH is set forth in SEQ ID NO: 17 or has at least 90% or at least 95% sequence identity thereto, and the VL is set forth in SEQ ID NO: 18 or has at least 90% or at least 95% sequence identity thereto;

the VH is set forth in SEQ ID NO: 19 or has at least 90% or at least 95% sequence identity thereto, and the VL is set forth in SEQ ID NO: 20;

the VH is set forth in SEQ ID NO: 21 or has at least 90% or at least 95% sequence identity thereto, and the VL is set forth in SEQ ID NO: 22 or has at least 90% or at least 95% sequence identity thereto; or

the VH is set forth in SEQ ID NO: 23 or has at least 90% or at least 95% sequence identity thereto, and the VL is set forth in SEQ ID NO: 24 or has at least 90% or at least 95% sequence identity thereto.

6. The anti-CD25 antibody or the antigen-binding fragment thereof according to any one of claims 1 to 5, wherein the antigen-binding fragment is an scFv, Fv, Fab or Fab' fragment.

7. The anti-CD25 antibody or the antigen-binding fragment thereof according to any one of claims 1 to 5, wherein the anti-CD25 antibody is an IgG antibody, preferably IgG1, IgG2 or IgG4;

preferably, the anti-CD25 antibody has a de-fucosylated site in the Fc region to enhance the binding ability to FcγRIIIa and/or to reduce the binding ability to FcγRIIb;
more preferably, the de-fucosylated site is position 297.

8. The anti-CD25 antibody or the antigen-binding fragment thereof according to any one of claims 1 to 7, comprising a heavy chain and a light chain, wherein:

1) the heavy chain is set forth in SEQ ID NO: 51 or has at least 80%, at least 90% or at least 95% sequence identity thereto, and the light chain is set forth in SEQ ID NO: 52 or has at least 80%, at least 90% or at least 95% sequence identity thereto;
2) the heavy chain is set forth in SEQ ID NO: 53 or has at least 80%, at least 90% or at least 95% sequence identity thereto, and the light chain is set forth in SEQ ID NO: 54 or has at least 80% identity thereto;
3) the heavy chain is set forth in SEQ ID NO: 55 or has at least 80%, at least 90% or at least 95% sequence identity thereto, and the light chain is set forth in SEQ ID NO: 56 or has at least 80%, at least 90% or at least 95% sequence identity thereto;
4) the heavy chain is set forth in SEQ ID NO: 57 or has at least 80%, at least 90% or at least 95% sequence identity thereto, and the light chain is set forth in SEQ ID NO: 58 or has at least 80%, at least 90% or at least 95% sequence identity thereto; or
5) the heavy chain is set forth in SEQ ID NO: 61 or has at least 80%, at least 90% or at least 95% sequence identity thereto, and the light chain is set forth in SEQ ID NO: 62 or has at least 80%, at least 90% or at least 95% sequence identity thereto.

9. The anti-CD25 antibody or the antigen-binding fragment thereof according to any one of claims 1 to 8, wherein the anti-CD25 antibody or the antigen-binding fragment thereof has at least one of the following characteristics:

(a) having a KD value for binding to human CD25 of less than $1\times10^{-7}$ M;
(b) having no inhibition or substantially no inhibition on the binding of IL-2 to CD25;
(c) depleting tumor-infiltrating Tregs, without affecting or substantially without affecting Teff function;
(d) binding to an Fcγ receptor with an activation/inhibition (A/I) rate greater than 1;
(e) binding to FcγRIIIa with higher affinity than binding to FcγRI, FcγRIIc and/or FcγRIIb, preferably, binding to FcγRIIIa with higher affinity than binding to FcγRIIb;
(f) inhibiting tumor growth; and
(g) triggering an enhanced CDC, ADCC and/or ADCP response; preferably, triggering an enhanced ADCC response.

10. An anti-CD25 antibody or an antigen-binding fragment thereof, competing for binding to human CD25, or competing for binding to or binding to the same epitope of human CD25, with the anti-CD25 antibody or the antigen-binding fragment thereof according to any one of claims 1 to 9.

11. A polynucleotide encoding the anti-CD25 antibody or the antigen-binding fragment thereof according to any one of claims 1 to 10.

12. A vector comprising the polynucleotide according to claim 11.

13. A host cell comprising the vector according to claim 12, wherein

preferably, the host cell is bacteria, yeast or a mammalian cell;
more preferably, the host cell is *Escherichia coli, Pichia pastoris,* a Chinese hamster ovary cell or a human embryonic kidney 293 cell.

14. A method for preparing an anti-CD25 antibody or an antigen-binding fragment thereof, comprising:

expressing the anti-CD25 antibody or the antigen-binding fragment thereof according to any one of claims 1 to 10 in the host cell according to claim 13, and
isolating the anti-CD25 antibody or the antigen-binding fragment thereof from the host cell.

**15.** A pharmaceutical composition, comprising:

the anti-CD25 antibody or the antigen-binding fragment thereof according to any one of claims 1 to 10; and one or more pharmaceutically acceptable excipients, diluents or carriers.

**16.** Use of any one or any combination thereof selected from the group consisting of the following in the preparation of a medicament or a pharmaceutical composition:
the anti-CD25 antibody or the antigen-binding fragment thereof according to any one of claims 1 to 10, the polynucleotide according to claim 11, and the pharmaceutical composition according to claim 15, wherein the medicament or pharmaceutical composition is used for treating cancer.

**17.** A method for treating a subject having cancer, comprising:
administering to the subject a therapeutically effective amount of the anti-CD25 antibody or the antigen-binding fragment thereof according to any one of claims 1 to 10, the polynucleotide according to claim 11, the pharmaceutical composition according to claim 15, or any combination thereof.

**18.** A method for reducing the number of and/or inhibiting the activity of intratumoral or tumor-infiltrating Treg cells in a subject, comprising:
administering to the subject a therapeutically effective amount of the anti-CD25 antibody or the antigen-binding fragment thereof according to any one of claims 1 to 10, the polynucleotide according to claim 11, the pharmaceutical composition according to claim 15, or any combination thereof.

**19.** A method for increasing the ratio of Teffs/Tregs in a tumor in a subject, comprising:
administering to the subject a therapeutically effective amount of the anti-CD25 antibody or the antigen-binding fragment thereof according to any one of claims 1 to 10, the polynucleotide according to claim 11, the pharmaceutical composition according to claim 15, or any combination thereof.

**20.** A method for enhancing CDC, ADCC and/or ADCP against a tumor cell in a subject, comprising:
administering to the subject a therapeutically effective amount of the anti-CD25 antibody or the antigen-binding fragment thereof according to any one of claims 1 to 10, the polynucleotide according to claim 11, the pharmaceutical composition according to claim 15, or any combination thereof.

**21.** The use according to claim 16 and the method according to any one of claims 17 to 20, wherein the subject has cancer or tumor,
preferably, the cancer or tumor is selected from the group consisting of squamous cell carcinoma, myeloma, small-cell lung cancer, non-small cell lung cancer, neuroglioma, hepatocellular carcinoma (HCC), Hodgkin's lymphoma, non-Hodgkin's lymphoma, acute myeloid leukemia (AML), multiple myelomas, gastrointestinal cancer, kidney cancer, ovarian cancer, liver cancer, lymphoblastic leukemia, lymphocytic leukemia, colorectal cancer, endometrial cancer, renal cancer, prostate cancer, thyroid cancer, melanoma, chondrosarcoma, neuroblastoma, pancreatic cancer, glioblastoma multiforme, cervical cancer, brain cancer, gastric cancer, bladder cancer, hepatoma, breast cancer, colon cancer, and head and neck cancer.

**22.** A method for detecting CD25 *in vivo* or *in vitro,* comprising using the anti-CD25 antibody or the antigen-binding fragment thereof according to any one of claims 1 to 10, and/or the polynucleotide according to claim 11, wherein preferably, the interaction between the anti-CD25 antibody or the antigen-binding fragment thereof, the polynucleotide and the CD25 is detected if CD25 is present.

**23.** A kit for detecting CD25, comprising the anti-CD25 antibody or the antigen-binding fragment thereof according to any one of claims 1 to 10, and/or the polynucleotide according to claim 11.

FIG. 1A

FIG. 1B

FIG. 2

FIG. 3A

FIG. 3B

FIG. 3C

FIG. 4A

FIG. 4B

FIG. 4C

FIG. 5A

FIG. 5B

FIG. 5C

FIG. 6A

FIG. 6B

FIG. 6C

FIG. 6D

FIG. 6E

FIG. 6F

FIG. 6G

FIG. 7A

FIG. 7B

FIG. 8

FIG. 9

**EP 4 151 655 A1**

FIG. 10

FIG. 11A

FIG. 11B

37

FIG. 12A

FIG. 12B

## INTERNATIONAL SEARCH REPORT

| International application No. |
|---|
| **PCT/CN2021/093791** |

### A. CLASSIFICATION OF SUBJECT MATTER

C07K 16/28(2006.01)i;  C07K 14/55(2006.01)i;  A61K 39/00(2006.01)i

According to International Patent Classification (IPC) or to both national classification and IPC

### B. FIELDS SEARCHED

Minimum documentation searched (classification system followed by classification symbols)

C07K; A61K

Documentation searched other than minimum documentation to the extent that such documents are included in the fields searched

Electronic data base consulted during the international search (name of data base and, where practicable, search terms used)

DWPI; VEN; SIPOABS; CNABS; 中国专利生物序列检索系统; CHINESE PATENT BIOLOGICAL SEQUENCE RETRIEVAL SYSTEM; CNKI; WANFANG; ISI web of Science; GenBank: CD25, IL-2RA, 肿瘤, 癌症, 抗体, 抗原结合, 信号, 抑制, 阻断, 调节T细胞, 受体, 申请人, applicants, tumor, cancer, antibody, antigen binding, signal, inhibit, block, Treg, receptor, SEQ ID NO:1-18

### C. DOCUMENTS CONSIDERED TO BE RELEVANT

| Category* | Citation of document, with indication, where appropriate, of the relevant passages | Relevant to claim No. |
|---|---|---|
| X | CN 110869388 A (TUSK THERAPEUTICS LTD. et al.) 06 March 2020 (2020-03-06) claims 1, 4-19, 21, 24, 25 and 48, and description, paragraphs [0008], [0013]-[0021], [0048], [0050], [0124], [0135]-[0137], [0150], [0160] and [0443]-[0473] | 10-23 |
| A | CN 111094346 A (TUSK THERAPEUTICS LTD. et al.) 01 May 2020 (2020-05-01) entire document | 1-23 |
| A | WO 2019008386 A1 (TUSK THERAPEUTICS LTD. et al.) 10 January 2019 (2019-01-10) entire document | 1-23 |
| A | WO 2019175215 A1 (TUSK THERAPEUTICS LTD. et al.) 19 September 2019 (2019-09-19) entire document | 1-23 |
| A | CN 105377892 A (ABBVIE BIOTECHNOLOGY LTD.) 02 March 2016 (2016-03-02) entire document | 1-23 |
| A | CN 101124244 A (GENMAB INC.) 13 February 2008 (2008-02-13) entire document | 1-23 |
| A | US 5530101 A (PROTEIN DESIGN LABS, INC.) 25 June 1996 (1996-06-25) entire document | 1-23 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 July 2021** | **18 August 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**EP 4 151 655 A1**

**INTERNATIONAL SEARCH REPORT**

| International application No. |
| --- |
| **PCT/CN2021/093791** |

**C.    DOCUMENTS CONSIDERED TO BE RELEVANT**

| Category* | Citation of document, with indication, where appropriate, of the relevant passages | Relevant to claim No. |
| --- | --- | --- |
| A | FLYNN, M.J. et al. "The Emerging Role of Anti-CD25 Directed Therapies as Both Immune Modulators and Targeted Agents in Cancer"<br>*British Journal of Haematology,* Vol. 179, 30 May 2017 (2017-05-30),<br>pp. 20-35 | 1-23 |
| A | GOUBIER, A. et al. "Non-IL-2 Blocking Anti-CD25-Targeting Antibodies: Depletion of Regulatory T-cells Driving Optimaleffector Response for Rejection of Established Tumors"<br>*Cancer Immunology Research,* Vol. 7, No. 2, 28 February 2019 (2019-02-28),<br>conference abstract A192 | 1-23 |

Form PCT/ISA/210 (second sheet) (January 2015)

<table>
<tr><td>**INTERNATIONAL SEARCH REPORT**</td><td>International application No.<br>**PCT/CN2021/093791**</td></tr>
</table>

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed:

          ☑  in the form of an Annex C/ST.25 text file.

          ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐  in the form of an Annex C/ST.25 text file (Rule 13*ter*.1(a)).

          ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT

International application No.

**PCT/CN2021/093791**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **17-20,21(部分)**
   because they relate to subject matter not required to be searched by this Authority, namely:

   [1] Claims 17-20 and 21 (in part) relate to methods of treating a subject with cancer, methods of reducing the subject's tumor Treg cells or their activity, methods of increasing the subject's internal Teff/Treg ratio, and methods of enhancing CDC, ADCC, and/or ADCP against tumor cells in the subject, which relate to methods of treatment of diseases by therapy as defined in PCT Rule 39.1(iv), and this report is made on the basis of the use of CD25 antibodies for cancer drug preparation.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.

**PCT/CN2021/093791**

| Patent document cited in search report | | | Publication date (day/month/year) | Patent family member(s) | | | Publication date (day/month/year) |
|---|---|---|---|---|---|---|---|
| CN | 110869388 | A | 06 March 2020 | EP | 3596123 | A1 | 22 January 2020 |
| | | | | WO | 2018167104 | A1 | 20 September 2018 |
| | | | | BR | 112019017017 | A2 | 14 April 2020 |
| | | | | IL | 269081 | D0 | 28 November 2019 |
| | | | | CA | 3056506 | A1 | 20 September 2018 |
| | | | | SG | 11201908578Y | A | 30 October 2019 |
| | | | | KR | 20190130137 | A | 21 November 2019 |
| | | | | US | 2020010554 | A1 | 09 January 2020 |
| | | | | CR | 20190477 | A | 07 January 2020 |
| | | | | JP | 2020514363 | A | 21 May 2020 |
| | | | | AU | 2018233976 | A1 | 31 October 2019 |
| | | | | CL | 2019002624 | A1 | 03 April 2020 |
| | | | | PE | 20191812 | A1 | 26 December 2019 |
| CN | 111094346 | A | 01 May 2020 | JP | 2020525517 | A | 27 August 2020 |
| | | | | EP | 3649153 | A1 | 13 May 2020 |
| | | | | US | 2020283535 | A1 | 10 September 2020 |
| | | | | WO | 2019008386 | A1 | 10 January 2019 |
| WO | 2019008386 | A1 | 10 January 2019 | JP | 2020525517 | A | 27 August 2020 |
| | | | | EP | 3649153 | A1 | 13 May 2020 |
| | | | | US | 2020283535 | A1 | 10 September 2020 |
| | | | | CN | 111094346 | A | 01 May 2020 |
| WO | 2019175215 | A1 | 19 September 2019 | US | 2021054084 | A1 | 25 February 2021 |
| | | | | BR | 112020016499 | A2 | 26 January 2021 |
| | | | | WO | 2019175226 | A1 | 19 September 2019 |
| | | | | EP | 3765511 | A1 | 20 January 2021 |
| | | | | US | 2019300613 | A1 | 03 October 2019 |
| | | | | US | 2019284287 | A1 | 19 September 2019 |
| | | | | SG | 11202008733Y | A | 29 October 2020 |
| | | | | US | 10745485 | B2 | 18 August 2020 |
| | | | | EP | 3765510 | A1 | 20 January 2021 |
| | | | | TW | 201938588 | A | 01 October 2019 |
| | | | | EP | 3765507 | A1 | 20 January 2021 |
| | | | | CN | 112020512 | A | 01 December 2020 |
| | | | | SG | 11202008784R | A | 29 October 2020 |
| | | | | PE | 20210289 | A1 | 11 February 2021 |
| | | | | US | 10738125 | B2 | 11 August 2020 |
| | | | | EP | 3765506 | A1 | 20 January 2021 |
| | | | | CN | 112218891 | A | 12 January 2021 |
| | | | | CN | 112041345 | A | 04 December 2020 |
| | | | | CR | 20200465 | A | 17 November 2020 |
| | | | | US | 2021009704 | A1 | 14 January 2021 |
| | | | | PE | 20210288 | A1 | 11 February 2021 |
| | | | | EP | 3765505 | A1 | 20 January 2021 |
| | | | | KR | 20200131861 | A | 24 November 2020 |
| | | | | CN | 112020514 | A | 01 December 2020 |
| | | | | US | 2021040221 | A1 | 11 February 2021 |
| | | | | US | 2021009703 | A1 | 14 January 2021 |
| | | | | SG | 11202008699W | A | 29 October 2020 |
| | | | | PE | 20210287 | A1 | 11 February 2021 |
| | | | | EP | 3765504 | A1 | 20 January 2021 |

Form PCT/ISA/210 (patent family annex) (January 2015)

**INTERNATIONAL SEARCH REPORT**
Information on patent family members

International application No.

**PCT/CN2021/093791**

| Patent document cited in search report | | | Publication date (day/month/year) | Patent family member(s) | | | Publication date (day/month/year) |
|---|---|---|---|---|---|---|---|
| | | | | CA | 3088659 | A1 | 19 September 2019 |
| | | | | CR | 20200467 | A | 17 November 2020 |
| | | | | US | 2021009702 | A1 | 14 January 2021 |
| | | | | WO | 2019175216 | A1 | 19 September 2019 |
| | | | | AU | 2019233581 | A1 | 03 September 2020 |
| | | | | TW | 202003034 | A | 16 January 2020 |
| | | | | EP | 3765503 | A1 | 20 January 2021 |
| | | | | CN | 112020516 | A | 01 December 2020 |
| | | | | US | 2021047420 | A1 | 18 February 2021 |
| | | | | US | 10752691 | B2 | 25 August 2020 |
| | | | | WO | 2019175220 | A1 | 19 September 2019 |
| | | | | WO | 2019175217 | A1 | 19 September 2019 |
| | | | | EP | 3765502 | A1 | 20 January 2021 |
| | | | | TW | 201940515 | A | 16 October 2019 |
| | | | | CA | 3088246 | A1 | 19 September 2019 |
| | | | | US | 2019322752 | A1 | 24 October 2019 |
| | | | | BR | 112020016519 | A2 | 15 December 2020 |
| | | | | AU | 2019233576 | A1 | 17 September 2020 |
| | | | | US | 2021009699 | A1 | 14 January 2021 |
| | | | | WO | 2019175222 | A1 | 19 September 2019 |
| CN | 105377892 | A | 02 March 2016 | AU | 2014233478 | A1 | 24 September 2015 |
| | | | | HK | 1220470 | A1 | 05 May 2017 |
| | | | | BR | 112015023084 | A2 | 21 November 2017 |
| | | | | WO | 2014145000 | A3 | 19 March 2015 |
| | | | | WO | 2014145000 | A2 | 18 September 2014 |
| | | | | US | 2020308290 | A1 | 01 October 2020 |
| | | | | JP | 2016515524 | A | 30 May 2016 |
| | | | | CA | 2904532 | A1 | 18 September 2014 |
| | | | | AU | 2014233478 | A8 | 22 October 2015 |
| | | | | US | 2015010539 | A1 | 08 January 2015 |
| | | | | MX | 2015012551 | A | 26 October 2016 |
| | | | | RU | 2015144026 | A | 20 April 2017 |
| | | | | EP | 3216804 | A3 | 20 December 2017 |
| | | | | EP | 3216804 | A2 | 13 September 2017 |
| | | | | EP | 2970488 | A2 | 20 January 2016 |
| | | | | US | 2017233481 | A1 | 17 August 2017 |
| CN | 101124244 | A | 13 February 2008 | CN | 101124244 | B | 10 October 2012 |
| | | | | WO | 2004045512 | A2 | 03 June 2004 |
| | | | | US | 2004170626 | A1 | 02 September 2004 |
| | | | | JP | 2006523433 | A | 19 October 2006 |
| | | | | WO | 2004045512 | A3 | 24 November 2005 |
| | | | | CA | 2505991 | A1 | 03 June 2004 |
| | | | | BR | PI0316282 | B1 | 30 April 2019 |
| | | | | PL | 217296 | B1 | 31 July 2014 |
| | | | | US | 2009081219 | A1 | 26 March 2009 |
| | | | | EP | 1578397 | A2 | 28 September 2005 |
| | | | | AU | 2003295471 | B2 | 25 August 2011 |
| | | | | KR | 20120056863 | A | 04 June 2012 |
| | | | | US | 2015259424 | A1 | 17 September 2015 |
| | | | | DK | 1578397 | T3 | 11 March 2013 |

Form PCT/ISA/210 (patent family annex) (January 2015)

**INTERNATIONAL SEARCH REPORT**
Information on patent family members

International application No.

**PCT/CN2021/093791**

| Patent document cited in search report | | | Publication date (day/month/year) | Patent family member(s) | | | Publication date (day/month/year) |
|---|---|---|---|---|---|---|---|
| | | | | EP | 1578397 | A4 | 09 May 2007 |
| | | | | US | 8182812 | B2 | 22 May 2012 |
| | | | | US | 9598493 | B2 | 21 March 2017 |
| | | | | KR | 20050086628 | A | 30 August 2005 |
| | | | | NO | 20052889 | L | 18 July 2005 |
| | | | | NZ | 540555 | A | 30 April 2008 |
| | | | | ES | 2401136 | T3 | 17 April 2013 |
| | | | | US | 2012244069 | A1 | 27 September 2012 |
| | | | | MX | PA05005160 | A | 22 July 2005 |
| | | | | UA | 90082 | C2 | 12 April 2010 |
| | | | | NO | 20052889 | A | 18 July 2005 |
| | | | | HK | 1077226 | A1 | 10 February 2006 |
| | | | | CA | 2505991 | C | 27 February 2018 |
| | | | | US | 7438907 | B2 | 21 October 2008 |
| | | | | EP | 1578397 | B1 | 26 December 2012 |
| | | | | NO | 20052889 | D0 | 14 June 2005 |
| | | | | PL | 377794 | A1 | 20 February 2006 |
| | | | | EA | 200500835 | A1 | 24 February 2006 |
| | | | | US | 2017240640 | A1 | 24 August 2017 |
| | | | | US | 10703818 | B2 | 07 July 2020 |
| | | | | IL | 168304 | A | 30 November 2010 |
| | | | | BR | 0316282 | A | 11 October 2005 |
| | | | | US | 8961968 | B2 | 24 February 2015 |
| | | | | KR | 101329843 | B1 | 14 November 2013 |
| | | | | AU | 2003295471 | A1 | 15 June 2004 |
| | | | | EA | 013677 | B1 | 30 June 2010 |
| | | | | JP | 4966497 | B2 | 04 July 2012 |
| US | 5530101 | A | 25 June 1996 | EP | 0566647 | B1 | 08 October 2003 |
| | | | | CA | 2098404 | C | 20 August 2002 |
| | | | | AT | 251639 | T | 15 October 2003 |
| | | | | JP | H06503963 | A | 12 May 1994 |
| | | | | KR | 100231090 | B1 | 15 November 1999 |
| | | | | US | 5693761 | A | 02 December 1997 |
| | | | | DE | 69133326 | T2 | 05 August 2004 |
| | | | | EP | 0566647 | A1 | 27 October 1993 |
| | | | | US | 6180370 | B1 | 30 January 2001 |
| | | | | AU | 671949 | B2 | 19 September 1996 |
| | | | | US | 5585089 | A | 17 December 1996 |
| | | | | EP | 0566647 | A4 | 14 June 1995 |
| | | | | EP | 1386932 | A1 | 04 February 2004 |
| | | | | WO | 9211018 | A1 | 09 July 1992 |
| | | | | JP | 3276369 | B2 | 22 April 2002 |
| | | | | AU | 9172691 | A | 22 July 1992 |
| | | | | DE | 69133326 | D1 | 13 November 2003 |
| | | | | AU | 7548196 | A | 20 February 1997 |
| | | | | US | 5693762 | A | 02 December 1997 |
| | | | | CA | 2098404 | A1 | 20 June 1992 |
| | | | | US | 7022500 | B1 | 04 April 2006 |

Form PCT/ISA/210 (patent family annex) (January 2015)

EP 4 151 655 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010408502
- WO 2004045512 A
- WO 2006108670 A
- WO 1993011238 A
- WO 1990007861 A
- WO 2017174331 A
- WO 2004074437 A
- WO 2006050172 A
- WO 2011077245 A
- WO 2016021720 A
- WO 2019175216 A
- WO 2018167104 A
- US 20030040606 A
- US 7494647 B
- WO 2009040562 A
- WO 2010035012 A
- WO 2005003169 A
- WO 2005003170 A
- WO 2005003171 A
- WO 9222583 A
- WO 05113605 A
- WO 0348731 A
- US 5530101 A
- US 20190284287 A

**Non-patent literature cited in the description**

- **MALEK et al.** *PNAS,* September 1983, vol. 80 (18), 5694-5698
- **ONIZUKA S et al.** *CancerRes.,* 01 July 1999, vol. 59 (13), 3128-3133
- **ZHANG et al.** *Cancer Biother Radiopharm.,* June 2009, vol. 24 (3), 303-309
- **YULIUS Y SETIADY et al.** *Eur J Immunol.,* March 2010, vol. 40 (3), 780-6
- Antibodies: A Laboratory Manual. Cold Spring Harbor Press
- *J. Biol. Chem,* 1968, vol. 243, 3558
- **JOHNSON ; WU.** *Nucleic Acids Res.,* 2000, vol. 28, 214-8
- **CHOTHIA et al.** *J. Mol. Biol.,* 1986, vol. 196, 901-17
- **CHOTHIA et al.** *Nature,* 1989, vol. 342, 877-83
- **MARTIN et al.** *Proc Natl Acad Sci (USA),* 1989, vol. 86, 9268-9272
- AbMTM, A Computer Program for Modeling Variable Regions of Antibodies. Oxford Molecular, Ltd
- **SAMUDRALA et al.** Ab Initio Protein Structure Prediction Using a Combined Hierarchical Approach. *PROTEINS, Structure, Function and Genetics Suppl.,* vol. 3, 194-198
- **MACCALLUM et al.** *J. Mol. Biol.,* 1996, vol. 5, 732-45
- **MAKABE et al.** *Journal of Biological Chemistry,* 2008, vol. 283, 1156-1166
- **HAYES J et al.** *J Inflamm Res,* 2016, vol. 9, 209-219
- **CLYNES et al.** *Proc Natl Acad Sci USA,* 1998, vol. 95, 652-6
- **LAZAR et al.** *Proc Natl Acad Sci USA,* 2006, vol. 103, 2005-2010
- **SMITH et al.** *Proc Natl Acad Sci USA,* 2012, vol. 109
- **RICHARDS et al.** *Mol Cancer Ther,* 2008, vol. 7, 2517-2527
- **HOLLIGER ; HUDSON.** *Nature Biotech.,* 2005, vol. 23 (9), 1126-1136
- **ADAIR ; LAWSON.** *Drug Design Reviews-Online,* 2005, vol. 2 (3), 209-217
- **VERMA et al.** *Journal of Immunological Methods,* 1998, vol. 216, 165-181
- **SAMBROOK et al.** Molecular Cloning: A Laboratory Manual,. Cold Spring Harbor Laboratory Press
- **AUSUBEL et al.** Current Protocols in Molecular Biology. Greene Publishing Association, Wiley Interscience
- **ZHANG et al.** *Cancer Biother Radiopharm,* 2009, vol. 24 (3), 303-309
- **MALEK.** *Immunology,* 1983, vol. 80, 5694-5698
- **ONIZUKA S et al.** *Canc Res.,* 1999, vol. 59, 3128-3133
- *Bioinformation,* 2014, vol. 10 (4), 180-186
- *Methods MolBiol.,* 2019, vol. 1904, 213-230